# EUROPEAN PATENT APPLICATION

(11) **EP 3 321 267 A1**
(43) Date of publication of application: **16.05.2018**
(21) Application number: 17020521.5
(22) Date of filing: 06.11.2017
(51) Int. Cl.: C07D 487/04

(54) **CRYSTALLINE FORMS OF 2-[1-ETHYLSULFONYL-3-[7H-PYRROLO[2,3-D]PYRIMIDIN-4-YL)PYRAZOL-1-YL]AZETIDIN-3-YL]ACETONITRILE SALTS AND PREPARATION THEREOF**

(30) Priority: 11.11.2016 CZ 20160705
(71) Applicant: Zentiva K.S., 102 37 Praha 10 (CZ)
(72) Inventor: Tieger, Eszter, H-9963 Magyarlak (HU); Tozickova, Hana, 155 00 Praha 5 (CZ); Tkadlecova, Marcela, 163 00 Praha 6 (CZ); Dammer, Ondrej, 253 01 Hostivice (CZ); Gurgut, Tomas, 142 00 Praha 12 (CZ)
(74) Representative: Jirotkova, Ivana

(57) **Abstract**

The solution relates to a salt which includes 2-[1-ethylsulfonyl-3-[4-(7H-pyrrolo[2,3-d]pyrimidin-4-yl)pyrazol-1-yl]-azetidin-3-yl]acetonitrile of formula I and at least one acidic component (HX) selected form the group consisting of hydrobromic acid, hydrochlorid acid, sulfuric acid, methanesulfonic acid, ethanesulfonic acid, p-toluenesulfonic acid, L-tartaric acid or fumaric acid, the acid addition salt being in the crystalline form.

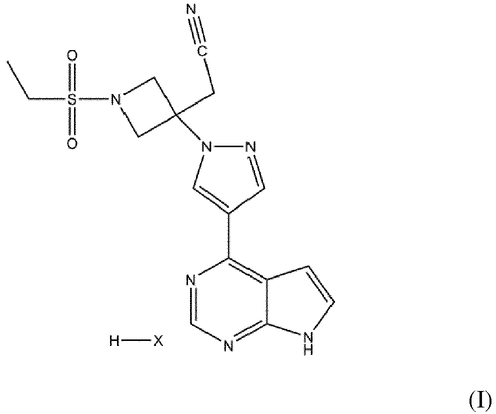

## Description

### Field of the invention

The present invention relates to crystalline forms of 2-[1-Ethylsulfonyl-3-[4-(7H-pyrrolo[2,3-d]pyrimidin-4-yl)pyrazol-1-yl]azetidin-3-yl]acetonitrile salts of Formula I, 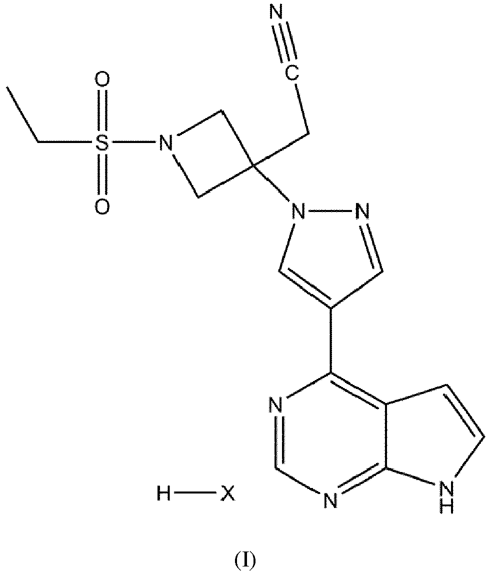 wherein HX represents at least one acid component, preferably HX is selected from the group consisting of hydrobromic acid, hydrochloric acid, sulfuric acid, methanesulfonic acid, ethanesulfonic acid, p-toluenesulfonic acid, L-tartaric acid and fumaric acid. The invention also relates to the processes for the preparation thereof as well as said use thereof in pharmaceutically acceptable compositions. Use of said crystalline forms of baricitinib and manufactured salts in the preparation of 2-[1-Ethylsulfonyl-3-[4-(7H-pyrrolo[2,3-d]pyrimidin-4-yl)pyrazol-1-yl]azetidin-3-yl]acetonitrile in the free form or in the form of any pharmaceutically acceptable salt thereof is also part of this invention.

### Background of the invention

2-[1-Ethylsulfonyl-3-[4-(7H-pyrrolo[2,3-d]pyrimidin-4-yl)pyrazol-1-yl]azetidin-3-yl]acetonitrile compound which is also known as baricitinib (CAS no.: 1187594-09-7) has a selective inhibitor activity on the Janus Associated Kinase 1 (JAK1) and Janus Associated Kinase 2 (JAK2) enzymes. It is a drug indicated for the treatment of rheumatoid arthritis and further investigated for the treatment of other diseases such as diabetic nephropathy, muscular degradation, dry eye disorders and psoriasis.

The enzymes Janus Associated Kinase 1 (JAK1) and Janus Associated Kinase 2 (JAK2) are non-receptor tyrosine kinases that mediate the signals via the JAK-STAT pathway. Cytokines play important roles in the control of the cell growth and the immune response. More specifically, Janus Associated Kinases are phosphorylate activated cytokine receptors recruiting STAT transcription factors which modulate gene transcription.

WO2009114512 describes protein kinase inhibitors with valuable pharmacological effect in the treatment of related diseases. One example of the compounds disclosed is 2-[1-Ethylsulfonyl-3-[4-(7H-pyrrolo[2,3-d]pyrimidin-4-yl)pyrazol-1-yl]azetidin-3-yl]acetonitrile. Preparation of the base is also described.

Salts of 2-[1-Ethylsulfonyl-3-[4-(7H-pyrrolo[2,3-d]pyrimidin-4-yl)pyrazol-1-yl]azetidin-3-yl]acetonitrile prepared with phosphoric acid and trifluoro acetic acid are also disclosed in WO2009114512. Following WO2015145286 discloses the amorphous form of baricitinib free form and WO2015166434 discloses a crystalline form thereof.

Many pharmaceutical solid compounds can exist in various crystalline forms regarded as polymorphs and hydrates/solvates having different crystal units and hence different physico-chemical properties including melting point, solubility, dissolution rate and finally, bioavailability. In order to distinguish the distinct solid phases of a compound several solid state analytical techniques can be used, *e.g.* X-Ray Powder Diffraction, solid state NMR and Raman spectroscopy, thermoanalytical methods.

Discovery of new solid phases (polymorphs, solvates and hydrates) of an active pharmaceutical compound offers the opportunity to select the appropriate modification having desirable physicochemical properties and processability and improve the characteristics of the pharmaceutical product. For this reason there is an explicit need for new solid forms (polymorphs, solvates, hydrates) of baricitinib and salts thereof especially in the crystalline form.

### Summary of the invention

The object of the present invention is to provide novel crystalline salts comprising 2-[1-Ethylsulfonyl-3-[4-(7H-pyrrolo[2,3-d]pyrimidin-4-yl)pyrazol-1-yl]azetidin-3-yl]acetonitrile of Formula I and at least one acid component (HX) suitable for oral administration which meet the pharmaceutical requirements. 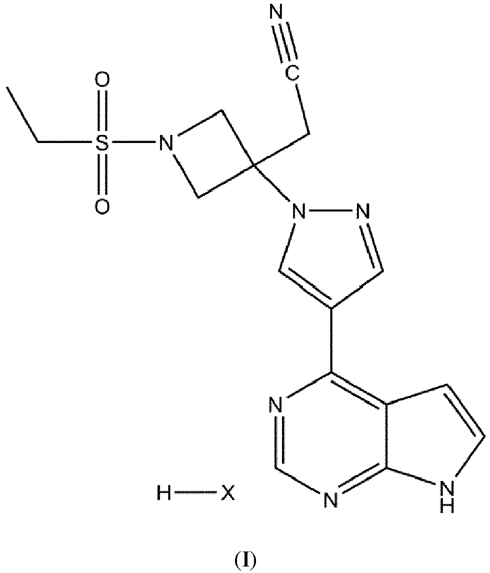 wherein HX represents at least one acid component, preferably hydrobromic acid, hydrochloric acid, sulfuric acid, methanesulfonic acid, ethanesulfonic acid, p-toluenesulfonic acid, L-tartaric acid, or fumaric acid.

In some embodiments of this invention, the solid forms are characterized by a variety of solid state analytical data, including for example X-ray powder diffraction pattern (XRPD) and differential scanning calorimetry (DSC) curve.

Provided is the Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrobromic acid salt of Formula I, wherein X represents Br, having an X-ray powder diffraction pattern comprising characteristic peaks at about 10.8; 16.8; 20.2; 21.1; 21.8; 25.7 and 27.8 ± 0.2° 2-theta measured by CuKα radiation. In some embodiments the Crystal modification 1 is characterised by differential scanning calorimetry curve having a melting process with T_{onset,1}=228.6°C; and Tₚₑₐₖ=243.4°C. In some embodiments the Crystal modification 1 is characterised by the thermal gravimetric curve having a 0.2% weigh loss in the range of 25°C to 226°C.

It should be understood that relative intensities can vary depending on a number of factors, including sample preparation, mounting, and the instrument and analytical procedure and settings used to obtain the spectrum.

Provided is a process for the preparation of the Crystal modification 1 wherein baricitinib free base is dissolved in an organic solvent by heating of the system to a temperature 50°C followed by the addition of the counterion, then kept at the temperature of 50°C for additional 1 hour and finally cooled back to room temperature. The solvent is selected from the group consisting of either polar protic solvents, preferably methanol, ethanol, 1-propanol, 2-propanol, 1-butanol, 2-butanol or a mixture thereof, or polar aprotic solvents, preferably acetone, acetonitrile, ethyl acetate, butyl acetate, tetrahydrofuran or a mixture thereof, more preferably tetrahydrofuran.

In some embodiments the process for the preparation of the Crystal modification 1 further comprises the steps of: a/ dissolution of baricitinib free base in the selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably in tetrahydrofuran, even more preferably tetrahydrofuran at the temperature of 50°C; b/ drop-wise addition of the hydrobromic acid 48% aqueous solution; c / cooling the solution of the step b/ to a temperature of 0-25°C while precipitation occurs; d/ keeping the suspension of the step c/ for about 16 hours at a temperature of 0-25°C ; e/ isolating the baricitinib hydrobromic acid salt in Crystal modification 1 and f/ optionally, drying of the product of step e/ at laboratory condition until the constant weight of the product is reached.

Provided is another process for the preparation of the Crystal modification 1, wherein baricitinib hydrobromic acid is suspended in 2-propanol at 50°C. In some embodiments the process for the preparation of the Crystal modification 1 further comprises the steps of: a/ suspending baricitinib hydrobromic acid salt in a solvent selected from the group consisting of: methanol, ethanol, 1-propanol, 2-propanol, 1-butanol, 2-butanol, acetone, acetonitrile, ethyl acetate, butyl acetate, tetrahydrofuran or a mixture thereof, more preferably in 2-propanol, even more preferably 2-propanol at 50°C; b/ stirring the suspension of step a/ at 50°C for 72 hours; c/ isolating the Crystal modification 1 of baricitinib hydrobromic acid salt and d/ optionally, drying of the product of step c/ at laboratory condition until the constant weight of the product is reached.

Provided is the Crystal modification 1 of 2-[1-Ethylsulfonyl-3-[4-(7H-pyrrolo[2,3-d]pyrimidin-4-yl)pyrazol-1-yllazetidin-3-yl]acetonitrile hydrochloric acid salt of Formula I, wherein X represents Cl, having an X-ray powder diffraction pattern comprising characteristic peaks at about 5.2; 8.9; 16.0; 20.0; 21.4; 23.9 and 28.7 ± 0.2° 2-thetameasured by CuKα radiation. In some embodiments the Crystal modification 1 is characterised by differential scanning calorimetry curve having a melting process with T_{onset,1}=208.4°C; and Tₚₑₐₖ=238.1°C. In some embodiments the Crystal modification 1 is characterised by the thermal gravimetric curve having a 0.35% weigh loss in the range of 25°C to 184°C.

It should be understood that relative intensities can vary depending on a number of factors, including sample preparation, mounting, and the instrument and analytical procedure and settings used to obtain the spectrum.

Provided is a process for the preparation of the Crystal modification 1 wherein baricitinib free base is dissolved in an organic solvent by heating of the system to a temperature 50°C followed by the addition of the counterion, then kept at the temperature of 50°C for additional 1 hour and finally cooled back to room temperature. The solvent is selected from the group consisting of either polar protic solvents, preferably methanol, ethanol, 1-propanol, 2-propanol, 1-butanol, 2-butanol or a mixture thereof, or polar aprotic solvents, preferably acetone, acetonitrile, ethyl acetate, butyl acetate, tetrahydrofuran or a mixture thereof, more preferably tetrahydrofuran.

In some embodiments the process for the preparation of the Crystal modification 1 further comprises the steps of: a/ dissolution of baricitinib free base in the selected solvent from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably in tetrahydrofuran, even more preferably tetrahydrofuran at the temperature of 50°C; b/ drop-wise addition of the hydrochloric acid 35% aqueous solution; c / cooling the solution of the step b/ to a temperature of 0-25°C while precipitation occurs; d/ keeping the suspension of the step c/ for about 16 hours at a temperature of 0-25°C ; e/ isolating the baricitinib hydrochloric acid salt in Crystal modification 1 and f/ optionally, drying of the product of step e/ at laboratory condition until the constant weight of the product is reached.

Provided is another process for the preparation of the Crystal modification 1, wherein baricitinib hydrobromic acid is suspended in acetone at 50°C. In some embodiments the process for the preparation of the Crystal modification 1 further comprises the steps of: a/ suspending baricitinib hydrochloric acid salt in a solvent selected from the group consisting of: methanol, ethanol, 1-propanol, 2-propanol, 1-butanol, 2-butanol, acetone, acetonitrile, ethyl acetate, butyl acetate, tetrahydrofuran or a mixture thereof, more preferably in acetone, even more preferably acetone at 50°C; b/ stirring the suspension of step a/ at 50°C for 72 hours; c/ isolating the Crystal modification 1 of baricitinib hydrochloric acid salt and d/ optionally, drying of the product of step c/ at laboratory condition until the constant weight of the product is reached.

Provided is the Crystal modification 2 of 2-[1-Ethylsulfonyl-3-[4-(7H-pyrrolo[2,3-d]pyrimidin-4-yl)pyrazol-1-yllazetidin-3-yl]acetonitrile hydrochloric acid salt of Formula I, wherein X represents Cl, having an X-ray powder diffraction pattern comprising characteristic peaks at about 11.1; 17.0; 20.2; 22.0; 24.5; 25.9 and 28.2 ± 0.2° 2-thetameasured by CuKα radiation. In some embodiments the Crystal modification 2 is characterised by differential scanning calorimetry curve having a melting process with Tₒₙₛₑₜ=214.73°C and Tₚₑₐₖ=235.27°C. In some embodiments the Crystal modification 2 is characterised by the thermal gravimetric curve having a 1.8% weigh loss in the range of 25°C-to 190°C.

It should be understood that relative intensities can vary depending on a number of factors, including sample preparation, mounting, and the instrument and analytical procedure and settings used to obtain the spectrum.

Provided is a process for the preparation of the Crystal modification 2, wherein baricitinib hydrochloric acid is suspended in water, preferably at 50°C.

In some embodiments the process for the preparation of the Crystal modification 2 further comprises the steps of: a/ suspending baricitinib hydrochloric acid salt in water at 50°C; b/ stirring the suspension of step a/ at 50°C for 72 hours; c/ isolating the Crystal modification 2 of baricitinib hydrochloric acid salt and d/ optionally, drying of the product of step c/ at laboratory condition until the constant weight of the product is reached.

Provided is the Crystal modification 1 of 2-[1-Ethylsulfonyl-3-[4-(7H-pyrrolo[2,3-d]pyrimidin-4-yl)pyrazol-1-yl]azetidin-3-yl]acetonitrile sulfuric acid salt of Formula I, wherein X represents HSO₄, having an X-ray powder diffraction pattern comprising characteristic peaks at about 8.8; 14.3; 17.7; 19.5; 20.8; 25.3 and 26.7 ± 0.2° 2-theta measured by CuKα radiation. In some embodiments the Crystal modification 1 is characterised by differential scanning calorimetry curve having a melting process with T_{onset,1}=206.0°C; and Tₚₑₐₖ=236.0°C. In some embodiments the Crystal modification 1 is characterised by the thermal gravimetric curve having a 0.8% weigh loss in the range of 25°C to 215°C.

It should be understood that relative intensities can vary depending on a number of factors, including sample preparation, mounting, and the instrument and analytical procedure and settings used to obtain the spectrum.

Provided is a process for the preparation of the Crystal modification 1 wherein baricitinib free base is dissolved in an organic solvent by heating of the system to a temperature 50°C followed by the addition of the counterion, then kept at the temperature of 50°C for additional 1 hour and finally cooled back to room temperature. The solvent is selected from the group consisting of either polar protic solvents, preferably methanol, ethanol, 1-propanol, 2-propanol, 1-butanol, 2-butanol or a mixture thereof, or polar aprotic solvents, preferably acetone, acetonitrile, ethyl acetate, butyl acetate, tetrahydrofuran or a mixture thereof, more preferably tetrahydrofuran.

In some embodiments the process for the preparation of the Crystal modification 1 further comprises the steps of: a/ dissolution of baricitinib free base in the selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably in tetrahydrofuran, even more preferably tetrahydrofuran at the temperature of 50°C; b/ drop-wise addition of the sulfuric acid 96% aqueous solution; c / cooling the solution of the step b/ to a temperature of 0-25°C while precipitation occurs; d/ keeping the suspension of the step c/ for about 16 hours at a temperature of 0-25°C ; e/ isolating the baricitinib sulfuric acid salt in Crystal modification 1 and f/ optionally, drying of the product of step e/ at laboratory condition until the constant weight of the product is reached.

Provided is another process for the preparation of the Crystal modification 1, wherein baricitinib sulfuric acid is suspended in ethyl acetate at 50°C. In some embodiments the process for the preparation of the Crystal modification 1 further comprises the steps of: a/ suspending baricitinib sulfuric acid salt in a solvent selected from the group consisting of: methanol, ethanol, 1-propanol, 2-propanol, 1-butanol, 2-butanol, acetone, acetonitrile, ethyl acetate, butyl acetate, tetrahydrofuran or a mixture thereof, more preferably in ethyl acetate, even more preferably ethyl acetate at 50°C; b/ stirring the suspension of step a/ at 50°C for 72 hours; c/ isolating the Crystal modification 1 of baricitinib sulfuric acid salt and d/ optionally, drying of the product of step c/ at laboratory condition until the constant weight of the product is reached.

Provided is the Crystal modification 1 of 2-[1-Ethylsulfonyl-3-[4-(7H-pyrrolo[2,3-d]pyrimidin-4-yl)pyrazol-1-yl]azetidin-3-yl]acetonitrile methanesulfonic acid salt of Formula I, wherein X represents CH₃SO₃, having an X-ray powder diffraction pattern comprising characteristic peaks at about 9.2; 15.3; 15.9; 18.6; 22.4; 25.4 and 28.8 ± 0.2° 2-theta measured by CuKα radiation. In some embodiments the Crystal modification 1 is characterised by differential scanning calorimetry curve having a melting process with T_{onset,1}=205.21°C; and Tₚₑₐₖ=221.5°C. In some embodiments the Crystal modification 1 is characterised by the thermal gravimetric curve having a 0.12% weigh loss in the range of 25°C to 215°C.

It should be understood that relative intensities can vary depending on a number of factors, including sample preparation, mounting, and the instrument and analytical procedure and settings used to obtain the spectrum.

Provided is the Crystal modification 1 of 2-[1-Ethylsulfonyl-3-[4-(7H-pyrrolo[2,3-d]pyrimidin-4-yl)pyrazol-1-yl]azetidin-3-yl]acetonitrile methanesulfonic acid salt of Formula I, having a unit cell, as determined by crystal X-ray diffraction, of the following dimensions: a=16.7330(2) Å, b=7.10319(6) Å, c=19.0201(3) Å, α=90°, β=115.817(17)°, γ=90°.

Provided is a process for the preparation of the Crystal modification 1 wherein baricitinib free base is dissolved in an organic solvent by heating of the system to a temperature 50°C followed by the addition of the counterion, then kept at the temperature of 50°C for additional 1 hour and finally cooled back to room temperature. The solvent is selected from the group consisting of either polar protic solvents, preferably methanol, ethanol, 1-propanol, 2-propanol, 1-butanol, 2-butanol or a mixture thereof, or polar aprotic solvents, preferably acetone, acetonitrile, ethyl acetate, butyl acetate, tetrahydrofuran or a mixture thereof, more preferably tetrahydrofuran.

In some embodiments the process for the preparation of the Crystal modification 1 further comprises the steps of: a/ dissolution of baricitinib free base in the selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably in tetrahydrofuran, even more preferably tetrahydrofuran at the temperature of 50°C; b/ drop-wise addition of the methanesulfonic acid >98% aqueous solution; c / cooling the solution of the step b/ to a temperature of 0-25°C while precipitation occurs; d/ keeping the suspension of the step c/ for about 16 hours at a temperature of 0-25°C ; e/ isolating the baricitinib methanesulfonic acid salt in Crystal modification 1 and f/ optionally, drying of the product of step e/ at laboratory condition until the constant weight of the product is reached.

Provided is another process for the preparation of the Crystal modification 1, wherein baricitinib methanesulfonic acid is suspended in methanol-water 1-1 V/V mixture at 50°C. In some embodiments the process for the preparation of the Crystal modification 1 further comprises the steps of: a/ suspending baricitinib methanesulfonic acid salt in a solvent selected from the group consisting of: water, methanol, ethanol, 1-propanol, 2-propanol, 1-butanol, 2-butanol, acetone, acetonitrile, ethyl acetate, butyl acetate, tetrahydrofuran or a mixture thereof, more preferably in methanol-water 1-1 V/V mixture, even more preferably methanol-water 1-1 V/V mixture at 50°C; b/ stirring the suspension of step a/ at 50°C for 72 hours; c/ isolating the Crystal modification 1 of baricitinib methanesulfonic acid salt and d/ optionally, drying of the product of step c/ at laboratory condition until the constant weight of the product is reached.

Provided is the Crystal modification 1 of 2-[1-Ethylsulfonyl-3-[4-(7H-pyrrolo[2,3-d]pyrimidin-4-yl)pyrazol-1-yl]azetidin-3-yl]acetonitrile ethanesulfonic acid salt of Formula I, wherein X represents C₂H₅SO₃. having an X-ray powder diffraction pattern comprising characteristic peaks at about 9.1; 15.3; 16.0; 18.3; 20.1 and 22:0 ± 0.2° 2-theta measured by CuKα radiation. In some embodiments the Crystal modification 1 is characterised by differential scanning calorimetry curve having a melting process with T_{onset,1}=165.1°C; and Tₚₑₐₖ=180.3°C. In some embodiments the Crystal modification 1 is characterised by the thermal gravimetric curve having a 0.5% weigh loss in the range of 25°C to 215°C.

It should be understood that relative intensities can vary depending on a number of factors, including sample preparation, mounting, and the instrument and analytical procedure and settings used to obtain the spectrum.

Provided is a process for the preparation of the Crystal modification 1 wherein baricitinib free base is dissolved in an organic solvent by heating of the system to a temperature 50°C followed by the addition of the counterion, then kept at the temperature of 50°C for additional 1 hour and finally cooled back to room temperature. The solvent is selected from the group consisting of either polar protic solvents, preferably methanol, ethanol, 1-propanol, 2-propanol, 1-butanol, 2-butanol or a mixture thereof, or polar aprotic solvents, preferably acetone, acetonitrile, ethyl acetate, butyl acetate, tetrahydrofuran or a mixture thereof, more preferably tetrahydrofuran.

In some embodiments the process for the preparation of the Crystal modification 1 further comprises the steps of: a/ dissolution of baricitinib free base in the selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably in tetrahydrofuran, even more preferably tetrahydrofuran at the temperature of 50°C; b/ drop-wise addition of the methanesulfonic acid >98% aqueous solution; c/ cooling the solution of the step b/ to a temperature of 0-25°C while precipitation occurs; d/ keeping the suspension of the step c/ for about 16 hours at a temperature of 0-25°C ; e/ isolating the baricitinib methanesulfonic acid salt in Crystal modification 1 and f/ optionally, drying of the product of step e/ at laboratory condition until the constant weight of the product is reached.

Provided is another process for the preparation of the Crystal modification 1, wherein baricitinib ethanesulfonic acid is suspended in ethyl acetate at 50°C. In some embodiments the process for the preparation of the Crystal modification 1 further comprises the steps of: a/ suspending baricitinib ethanesulfonic acid salt in a solvent selected from the group consisting of: water, methanol, ethanol, 1-propanol, 2-propanol, 1-butanol, 2-butanol, acetone, acetonitrile, ethyl acetate, butyl acetate, tetrahydrofuran or a mixture thereof, more preferably ethyl acetate, even more preferably ethyl acetate at 50°C; b/ stirring the suspension of step a/ at 50°C for 72 hours; c/ isolating the Crystal modification 1 of baricitinib ethanesulfonic acid salt and d/ optionally, drying of the product of step c/ at laboratory condition until the constant weight of the product is reached.

Provided is the Crystal modification 1 of 2-[1-Ethylsulfonyl-3-[4-(7H-pyrrolo[2,3-d]pyrimidin-4-yl)pyrazol-1-yllazetidin-3-yl]acetonitrile p-toluenesulfonic acid salt of Formula I, wherein X represents CH₃C₆H₄SO₃. having an X-ray powder diffraction pattern comprising characteristic peaks at about 5.8; 11.5; 17.6; 20.2; 23.1 and 28.0 ± 0.2° 2-theta measured by CuKα radiation. In some embodiments the Crystal modification 1 is characterised by differential scanning calorimetry curve having a melting process with T_{onset,1}=212.6°C; and Tₚₑₐₖ=223.8°C. In some embodiments the Crystal modification 1 is characterised by the thermal gravimetric curve having a 0.18% weigh loss in the range of 25°C to 240°C.

Provided is the Crystal modification 1 of 2-[1-Ethylsulfonyl-3-[4-(7H-pyrrolo[2,3-d]pyrimidin-4-yl)pyrazol-1-yllazetidin-3-yl]acetonitrile p-toluenesulfonic acid salt of Formula I, having a unit cell, as determined by crystal X-ray diffraction, of the following dimensions: a=9.0391(13) Å, b=30.7340(3) Å, c=9.7357(15) Å, α=90°, β=115.244(18) °, γ=90°.

It should be understood that relative intensities can vary depending on a number of factors, including sample preparation, mounting, and the instrument and analytical procedure and settings used to obtain the spectrum.

Provided is a process for the preparation of the Crystal modification 1 wherein baricitinib free base is dissolved in an organic solvent by heating of the system to a temperature 50°C followed by the addition of the counterion, then kept at the temperature of 50°C for additional 1 hour and finally cooled back to room temperature. The solvent is selected from the group consisting of either polar protic solvents, preferably methanol, ethanol, 1-propanol, 2-propanol, 1-butanol, 2-butanol or a mixture thereof, or polar aprotic solvents, preferably acetone, acetonitrile, ethyl acetate, butyl acetate, tetrahydrofuran or a mixture thereof, more preferably tetrahydrofuran.

In some embodiments the process for the preparation of the Crystal modification 1 further comprises the steps of: a/ dissolution of baricitinib free base in the selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably in tetrahydrofuran, even more preferably tetrahydrofuran at the temperature of 50°C; b/ drop-wise addition of the p-toluenesulfonic acid aqueous solution; c / cooling the solution of the step b/ to a temperature of 0-25°C while precipitation occurs; d/ keeping the suspension of the step c/ for about 16 hours at a temperature of 0-25°C ; e/ isolating the baricitinib p-toluenesulfonic acid salt in Crystal modification 1 and f/ optionally, drying of the product of step e/ at laboratory condition until the constant weight of the product is reached.

Provided is another process for the preparation of the Crystal modification 1, wherein baricitinib p-toluenesulfonic acid is suspended in tetrahydrofuran-water 95-5 V/V mixture at 50°C. In some embodiments the process for the preparation of the Crystal modification 1 further comprises the steps of: a/ suspending baricitinib p-toluenesulfonic acid salt in a solvent selected from the group consisting of: water, methanol, ethanol, 1-propanol, 2-propanol, 1-butanol, 2-butanol, acetone, acetonitrile, ethyl acetate, butyl acetate, tetrahydrofuran or a mixture thereof, more preferably in tetrahydrofuran-water 95-5 V/V mixture, even more preferably tetrahydrofuran-water 95-5 V/V mixture at 50°C; b/ stirring the suspension of step a/ at 50°C for 72 hours; c/ isolating the Crystal modification 1 of baricitinib p-toluenesulfonic acid salt and d/ optionally, drying of the product of step c/ at laboratory condition until the constant weight of the product is reached.

Provided is the Crystal modification 2 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt of Formula I, wherein X represents CH₃C₆H₄SO₃. having an X-ray powder diffraction pattern comprising characteristic peaks at about 5.8; 10.2; 17.1; 18.2; 22.3 and 27.5 ± 0.2° 2-theta measured by CuKα radiation. In some embodiments the Crystal modification 2 is characterised by differential scanning calorimetry curve having a melting process with T_{onset,1}=198.9°C; and Tₚₑₐₖ=221.8°C. In some embodiments the Crystal modification 1 is characterised by the thermal gravimetric curve having a 0.25% weigh loss in the range of 25°C to 234°C.

It should be understood that relative intensities can vary depending on a number of factors, including sample preparation, mounting, and the instrument and analytical procedure and settings used to obtain the spectrum.

Provided is a process for the preparation of the Crystal modification 2, wherein baricitinib p-toluenesulfonic acid is suspended in an organic solvent by heating of the system to a temperature 50°C. The solvent is selected from the group consisting of either polar protic solvents, preferably methanol, ethanol, 1-propanol, 2-propanol, 1-butanol, 2-butanol or a mixture thereof, or polar aprotic solvents, preferably acetone, acetonitrile, ethyl acetate, butyl acetate, tetrahydrofuran or a mixture thereof, more preferably methanol.

In some embodiments the process for the preparation of the Crystal modification 2 further comprises the steps of: a/ suspending baricitinib p-toluenesulfonic acid salt in a solvent selected from the group consisting of: water, methanol, ethanol, 1-propanol, 2-propanol, 1-butanol, 2-butanol, acetone, acetonitrile, ethyl acetate, butyl acetate, tetrahydrofuran or a mixture thereof, more preferably in methanol, even more preferably methanol at 50°C; b/ stirring the suspension of step a/ at 50°C for 72 hours; c/ isolating the Crystal modification 2 of baricitinib p-toluenesulfonic acid salt and d/ optionally, drying of the product of step c/ at laboratory condition until the constant weight of the product is reached.

Provided is the Crystal modification 1 of 2-[1-Ethylsulfonyl-3-[4-(7H-pyrrolo[2,3-d]pyrimidin-4-yl)pyrazol-1-yl]azetidin-3-yl]acetonitrile fumaric acid salt of Formula I, wherein X represents C₄H₃O₄, having an X-ray powder diffraction pattern comprising characteristic peaks at about 7.7; 10.1; 14.9; 19.1; 21.2; 25.5 and 27.6 ± 0.2° 2-theta measured by CuKα radiation. In some embodiments the Crystal modification 1 is characterised by differential scanning calorimetry curve having a melting process with T_{onset,1}=188.5°C; and Tₚₑₐₖ=212.6°C. In some embodiments the Crystal modification 1 is characterised by the thermal gravimetric curve having a 0.2% weigh loss in the range of 25°C to 172°C.

It should be understood that relative intensities can vary depending on a number of factors, including sample preparation, mounting, and the instrument and analytical procedure and settings used to obtain the spectrum.

Provided is a process for the preparation of the Crystal modification 1 wherein baricitinib free base is dissolved in an organic solvent by heating of the system to a temperature 50°C followed by the addition of the counterion, then kept at the temperature of 50°C for additional 1 hour and finally cooled back to room temperature. The solvent is selected from the group consisting of either polar protic solvents, preferably methanol, ethanol, 1-propanol, 2-propanol, 1-butanol, 2-butanol or a mixture thereof, or polar aprotic solvents, preferably acetone, acetonitrile, ethyl acetate, butyl acetate, tetrahydrofuran or a mixture thereof, more preferably tetrahydrofuran.

In some embodiments the process for the preparation of the Crystal modification 1 further comprises the steps of: a/ dissolution of baricitinib free base in the selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably in tetrahydrofuran, even more preferably tetrahydrofuran at the temperature of 50°C; b/ drop-wise addition of the fumaric acid aqueous solution; c / cooling the solution of the step b/ to a temperature of 0-25°C while precipitation occurs; d/ keeping the suspension of the step c/ for about 16 hours at a temperature of 0-25°C ; e/ isolating the baricitinib fumaric acid salt in Crystal modification 1 and f/ optionally, drying of the product of step e/ at laboratory condition until the constant weight of the product is reached.

Provided is another process for the preparation of the Crystal modification 1, wherein baricitinib fumaric acid is suspended in ethyl acetate at 50°C. In some embodiments the process for the preparation of the Crystal modification 1 further comprises the steps of: a/ suspending baricitinib fumaric acid salt in a solvent selected from the group consisting of: methanol, ethanol, 1-propanol, 2-propanol, 1-butanol, 2-butanol, acetone, acetonitrile, ethyl acetate, butyl acetate, tetrahydrofuran or a mixture thereof, more preferably in ethyl acetate, even more preferably ethyl acetate at 50°C; b/ stirring the suspension of step a/ at 50°C for 72 hours; c/ isolating the Crystal modification 1 of baricitinib fumaric acid salt and d/ optionally, drying of the product of step c/ at laboratory condition until the constant weight of the product is reached.

Provided is the Crystal modification 1 of 2-[1-Ethylsulfonyl-3-[4-(7H-pyrrolo[2,3-d]pyrimidin-4-yl)pyrazol-1-yl]azetidin-3-yl]acetonitrile L-tartaric acid salt of Formula I, wherein X represents C₄H₅O₆, having an X-ray powder diffraction pattern comprising characteristic peaks at about 10.8; 14.0; 17.8; 19.4; 21.6 and 25.4 ± 0.2° 2-theta measured by CuKα radiation. In some embodiments the Crystal modification 1 is characterised by differential scanning calorimetry curve having a melting process with T_{onset,1}=173.1°C; and Tₚₑₐₖ=181.1°C. In some embodiments the Crystal modification 1 is characterised by the thermal gravimetric curve having a 0.1% weigh loss in the range of 25°C to 172°C.

It should be understood that relative intensities can vary depending on a number of factors, including sample preparation, mounting, and the instrument and analytical procedure and settings used to obtain the spectrum.

Provided is a process for the preparation of the Crystal modification 1 wherein baricitinib free base is dissolved in an organic solvent by heating of the system to a temperature 50°C followed by the addition of the counterion, then kept at the temperature of 50°C for additional 1 hour and finally cooled back to room temperature. The solvent is selected from the group consisting of either polar protic solvents, preferably methanol, ethanol, 1-propanol, 2-propanol, 1-butanol, 2-butanol or a mixture thereof, or polar aprotic solvents, preferably acetone, acetonitrile, ethyl acetate, butyl acetate, tetrahydrofuran or a mixture thereof, more preferably tetrahydrofuran.

In some embodiments the process for the preparation of the Crystal modification 1 further comprises the steps of: a/ dissolution of baricitinib free base in the selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably in tetrahydrofuran, even more preferably tetrahydrofuran at the temperature of 50°C; b/ drop-wise addition of the L-tartaric acid aqueous solution; c / cooling the solution of the step b/ to a temperature of 0-25°C while precipitation occurs; d/ keeping the suspension of the step c/ for about 16 hours at a temperature of 0-25°C ; e/ isolating the baricitinib fumaric acid salt in Crystal modification 1 and f/ optionally, drying of the product of step e/ at laboratory condition until the constant weight of the product is reached.

Provided is another process for the preparation of the Crystal modification 1, wherein baricitinib L-tartaric acid is suspended in acetone at 50°C. In some embodiments the process for the preparation of the Crystal modification 1 further comprises the steps of: a/ suspending baricitinib L-tartaric acid salt in a solvent selected from the group consisting of: methanol, ethanol, 1-propanol, 2-propanol, 1-butanol, 2-butanol, acetone, acetonitrile, ethyl acetate, butyl acetate, tetrahydrofuran or a mixture thereof, more preferably in acetone, even more preferably acetone at 50°C; b/ stirring the suspension of step a/ at 50°C for 72 hours; c/ isolating the Crystal modification 1 of baricitinib L-tartaric acid salt and d/ optionally, drying of the product of step c/ at laboratory condition until the constant weight of the product is reached.

It should be understood that relative intensities can vary depending on a number of factors, including sample preparation, mounting, and the instrument and analytical procedure and settings used to obtain the spectrum.

Provided is a process for the preparation the amorphous phase of the citric acid salt of baricitinib wherein baricitinib free base is dissolved in an organic solvent by heating of the system to a temperature 50°C followed by the addition of the counterion, then kept at the temperature of 50°C for additional 1 hour and finally cooled back to room temperature. After keeping the solution at room temperature for 2 hours the solvent is evaporated till complete dryness. The solvent is selected from the group consisting of either polar protic solvents, preferably methanol, ethanol, 1-propanol, 2-propanol, 1-butanol, 2-butanol or a mixture thereof, or polar aprotic solvents, preferably acetone, acetonitrile, ethyl acetate, butyl acetate, tetrahydrofuran or a mixture thereof, more preferably tetrahydrofuran.

In some embodiments the process for the preparation of the amorphous phase of the citric acid salt further comprises the steps of: a/ dissolving baricitinib free base in tetrahydrofuran at temperature of 50°C; b/ addition of citric acid aqueous solution to the solution of step a/; c/ agitation of the solution of step b/ at 50°C for additional 60 minutes; d/ cooling the solution of the step c/ to room temperature; e/ keeping the solution of the step d/ for 2 hours at room temperature; f/ evaporating the solvent completely of the solution of step e/ and g/ isolating the baricitinib citric acid salt in amorphous phase.

The present invention further relates to the use of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrobromic acid salt of Formula I, wherein X represents Br, for the preparation of a pharmaceutical composition.

In another embodiment the present invention further relates to the use of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt of Formula I, wherein X represents Cl, for the preparation of a pharmaceutical composition.

In yet another embodiment the present invention further relates to the use of the Crystal modification 2 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt of Formula I, wherein X represents Cl, for the preparation of a pharmaceutical composition.

In another embodiment the present invention further relates to the use of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile sulfuric acid salt of Formula I, wherein X represents HSO₄, for the preparation of a pharmaceutical composition.

In another embodiment the present invention further relates to the use of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile methanesulfonic acid salt of Formula I, wherein X represents CH₃SO₃, for the preparation of a pharmaceutical composition.

In another embodiment the present invention further relates to the use of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile ethanesulfonic acid salt of Formula I, wherein X represents C₂H₅SO₃, for the preparation of a pharmaceutical composition.

In another embodiment the present invention further relates to the use of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt of Formula I, wherein X represents CH₃C₆H₄SO₃, for the preparation of a pharmaceutical composition.

In another embodiment the present invention further relates to the use of the Crystal modification 2 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt of Formula I, wherein X represents CH₃C₆H₄SO₃, for the preparation of a pharmaceutical composition.

In another embodiment the present invention further relates to the use of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile fumaric acid salt of Formula I, wherein X represents C₄H₃O₄, for the preparation of a pharmaceutical composition.

In another embodiment the present invention further relates to the use of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile L-tartaric acid salt of Formula I, wherein X represents C₄H₅O₆, for the preparation of a pharmaceutical composition.

In yet another embodiment the present invention further relates to a pharmaceutical formulations containing one or more solid forms of 2-[1-Ethylsulfonyl-3-[4-(7H-pyrrolo[2,3-d]pyrimidin-4-yl)pyrazol-1-yllazetidin-3-yl]acetonitrile hydrobromic acid, 2-[1-Ethylsulfonyl-3-[4-(7H-pyrrolo[2,3-d]pyrimidin-4-yl)pyrazol-1-yl]azetidin-3-yl]acetonitrile hydrochloric acid, 2-[1-Ethylsulfonyl-3-[4-(7H-pyrrolo[2,3-d]pyrimidin-4-yl)pyrazol-1-yl]azetidin-3-yl]acetonitrile sulfuric acid 2-[1-Ethylsulfonyl-3-[4-(7H-pyrrolo[2,3-d]pyrimidin-4-yl)pyrazol-1-yl]azetidin-3-yl]acetonitrile methanesulfonic acid, 2-[1-Ethylsulfonyl-3-[4-(7H-pyrrolo[2,3-d]pyrimidin-4-yl)pyrazol-1-yl]azetidin-3-yl]acetonitrile ethanesulfonic acid, 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid, 2-[1-Ethylsulfonyl-3-[4-(7H-pyrrolo[2,3-d]pyrimidin-4-yl)pyrazol-1-yl]azetidin-3-yl]acetonitrile fumaric acid, 2-[1-Ethylsulfonyl-3-[4-(7H-pyrrolo[2,3-d]pyrimidin-4-yl)pyrazol-1-yl]azetidin-3-yl]acetonitrile L-tartaric acid, and 2-[1-Ethylsulfonyl-3-[4-(7H-pyrrolo[2,3-d]pyrimidin-4-yl)pyrazol-1-yl]azetidin-3-yl]acetonitrile citric acid and a pharmaceutically acceptable carrier for the use thereof for the treatment of rheumatoid arthritis.

The present invention further relates to a pharmaceutical formulations containing the modifications of 2-[1-Ethylsulfonyl-3-[4-(7H-pyrrolo[2,3-d]pyrimidin-4-yl)pyrazol-1-yl]azetidin-3-yl]acetonitrile hydrobromic acid, hydrochloric acid, sulfuric, methanesulfonic, ethanesulfonic, p-toluenesulfonic, fumaric, L-tartaric or citric salts and the use thereof for the treatment of rheumatoid arthritis.

### Brief description of the figures

The figures depict the following spectrum, patterns and curves the various solid phases prepared according to the present invention.
**Figure 1** is an XRPD pattern of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrobromic acid salt;
**Figure 2** is an FTIR spectrum of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrobromic acid salt;
**Figure 3** is a Raman spectrum of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrobromic acid salt
**Figure 4** is a DSC curve of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrobromic acid salt;
**Figure 5** is a TGA curve of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrobromic acid salt;
**Figure 6** is an XRPD pattern of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt;
**Figure 7** is an FTIR spectrum of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt;
**Figure 8** is a Raman spectrum of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt;
**Figure 9** is a DSC curve of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt;
**Figure 10** is a TGA curve of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt;
**Figure 11** is an XRPD pattern of the Crystal modification 2 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt;
**Figure 12** is an - FTIR spectrum of the Crystal modification 2 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt;
**Figure 13** is a Raman spectrum of the Crystal modification 2 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt;
**Figure 14** is a DSC curve of the Crystal modification 2 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt;
**Figure 15** is a TGA curve of the Crystal modification 2 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt;
**Figure 16** is an XRPD pattern of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile sulfuric acid salt;
**Figure 17** is an FTIR spectrum of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile sulfuric acid salt;
**Figure 18** is a Raman spectrum of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile sulfuric acid salt;
**Figure 19** is a DSC curve of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile sulfuric acid salt;
**Figure 20** is a TGA curve of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile sulfuric acid salt;
**Figure 21** is an XRPD pattern of the Crystal modification 1of 2-[1-Ethylsulfonyl-3-[4-(7H-pyrrolo[2,3-d]pyrimidin-4-yl)pyrazol-1-yl]azetidin-3-yl]acetonitrile methanesulfonic acid salt;
**Figure 22** is an FTIR spectrum of the Crystal modification 1of 2-[1-Ethylsulfonyl-3-[4-(7H-pyrrolo[2,3-d]pyrimidin-4-yl)pyrazol-1-yl]azetidin-3-yl]acetonitrile methanesulfonic acid salt;
**Figure 23** is a Raman spectrum of the Crystal modification 1of 2-[1-Ethylsulfonyl-3-[4-(7H-pyrrolo[2,3-d]pyrimidin-4-yl)pyrazol-1-yl]azetidin-3-yl]acetonitrile methanesulfonic acid salt;
**Figure 24** is a DSC curve of the Crystal modification 1of 2-[1-Ethylsulfonyl-3-[4-(7H-pyrrolo[2,3-d]pyrimidin-4-yl)pyrazol-1-yl]azetidin-3-yl]acetonitrile methanesulfonic acid salt;
**Figure 25** is a TGA curve of the Crystal modification 1of 2-[1-Ethylsulfonyl-3-[4-(7H-pyrrolo[2,3-d]pyrimidin-4-yl)pyrazol-1-yl]azetidin-3-yl]acetonitrile methanesulfonic acid salt;
**Figure 26** is an ¹H-NMR spectrum of Crystal modification 1of 2-[1-Ethylsulfonyl-3-[4-(7H-pyrrolo[2,3-d]pyrimidin-4-yl)pyrazol-1-yl]azetidin-3-yl]acetonitrile methanesulfonic acid salt;
**Figure 27** is an XRPD pattern of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile ethanesulfonic acid salt;
**Figure 28** is an FTIR spectrum of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile ethanesulfonic acid salt;
**Figure 29** is a Raman spectrum of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile ethanesulfonic acid salt;
**Figure 30** is a DSC curve of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile ethanesulfonic acid salt;
**Figure 31** is a TGA curve of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile ethanesulfonic acid salt;
**Figure 32** is an ¹H-NMR spectrum of Crystal modification 1 of 2-[1-Ethylsulfonyl-3-[4-(7H-pyrrolo[2,3-d]pyrimidin-4-yl)pyrazol-1-yl]azetidin-3-yl]acetonitrile ethanesulfonic acid salt;
**Figure 33** is an XRPD pattern of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt;
**Figure 34** is an - FTIR spectrum of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt;
**Figure 35** is a Raman spectrum of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt
**Figure 36** is a DSC curve of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt;
**Figure 37** is a TGA curve of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt;
**Figure 38** is a ¹H-NMR spectrum of Crystal modification 1 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt;
**Figure 39** is an XRPD pattern of the Crystal modification 2 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt;

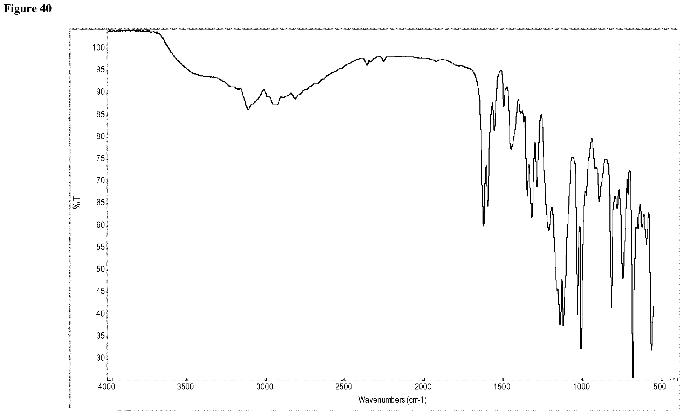

**Figure 40** is an FTIR spectrum of the Crystal modification 2 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt;
**Figure 41** is a Raman spectrum of the Crystal modification 2 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt;
**Figure 42** is a DSC curve of the Crystal modification 2 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt;
**Figure 43** is a TGA curve of the Crystal modification 2 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt;
**Figure 44** is a ¹H-NMR spectrum of Crystal modification 2 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt;
**Figure 45** is an XRPD pattern of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile fumaric acid salt;
**Figure 46** is an FTIR spectrum of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile fumaric acid salt;
**Figure 47** is a Raman spectrum of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile fumaric acid salt;
**Figure 48** is a DSC curve of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile fumaric acid salt;
**Figure 49** is a TGA curve of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile fumaric acid salt;
**Figure 50** is an ¹H-NMR spectrum of Crystal modification 1 of 2-[1-Ethylsulfonyl-3-[4-(7H-pyrrolo[2,3-d]pyrimidin-4-yl)pyrazol-1-yl]azetidin-3-yl]acetonitrile fumaric acid salt
**Figure 51** is an XRPD pattern of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile L-tartaric acid salt;
**Figure 52** is an FTIR spectrum of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile L-tartaric acid salt;
**Figure 53** is a Raman spectrum of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile L-tartaric acid salt;
**Figure 54** is a DSC curve of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile L-tartaric acid salt;
**Figure 55** is a TGA curve of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile L-tartaric acid salt;
**Figure 56** is an ¹H-NMR spectrum of Crystal modification 1 of 2-[1-Ethylsulfonyl-3-[4-(7H-pyrrolo[2,3-d]pyrimidin-4-yl)pyrazol-1-yl]azetidin-3-yl]acetonitrile L-tartaric acid salt
**Figure 57** is an XRPD pattern of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile citric acid salt;
**Figure 58** is an FTIR spectrum of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile citric acid salt;
**Figure 59** is a Raman spectrum of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile citric acid salt;
**Figure 60** is a DSC curve of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile citric acid salt;
**Figure 61** is an ¹H-NMR spectrum of Crystal modification 1 of 2-[1-Ethylsulfonyl-3-[4-(7H-pyrrolo[2,3-d]pyrimidin-4-yl)pyrazol-1-yl]azetidin-3-yl]acetonitrile citric acid salt

### Detailed description of the invention

The following description is presented to enable a person of ordinary skill in the art to make and use the various embodiments. Descriptions of specific devices, techniques, and applications are provided only as examples. Various modifications to the examples described herein will be readily apparent to those of ordinary skill in the art, and the general principles described herein may be applied to other examples and applications without departing from the spirit and scope of the various embodiments. Therefore, the various embodiments are not intended to be limited to the examples described herein and shown, but are to be accorded the scope consistent with the claims.

The aim of the present invention is to provide novel crystalline forms of 2-[1-Ethylsulfonyl-3-[4-(7H-pyrrolo[2,3-d]pyrimidin-4-yl)pyrazol-1-yl]azetidin-3-yl]acetonitrile salts of Formula I, 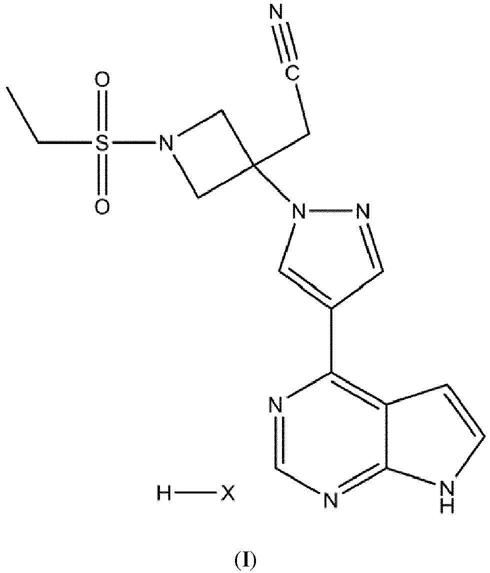 wherein HX represents at least one acid component, preferably HX is selected from the group consisting of hydrobromic acid, hydrochloric acid, sulfuric acid, methanesulfonic acid, ethanesulfonic acid, p-toluenesulfonic acid, L-tartaric acid, or fumaric acid, with advantageous properties for pharmaceutical use regarding the physico-chemical properties and can be produced in a reproducible manner even in industrial scale. The invention also relates to the processes for the preparation thereof as well as said use thereof in pharmaceutically acceptable compositions. Use of said crystalline forms of baricitinib and manufactured salts in the preparation of 2-[1-Ethylsulfonyl-3-[4-(7H-pyrrolo[2,3-d]pyrimidin-4-yl)pyrazol-1-yl]azetidin-3-yl]acetonitrile in the free form or in the form of any pharmaceutically acceptable salt thereof is also part of this invention.

Variations in the crystal structure of baricitinib salts may affect the dissolution rate (which may affect bioavailability etc.), manufacturability (*e.g*., ease of handling, ability to consistently prepare doses of known strength) and stability (*e.g*., thermal stability, shelf life, etc.) of a pharmaceutical drug product, particularly when formulated in a solid oral dosage form (*e.g*., in a form of a tablet). The therapeutic use and manufacturing of ibaricitinib involves the development of a new solid form of baricitinib salts that is more bioavailable and stable.

The term "modification, modifications" of baricitinib, as used in this document, is synonymous to terms "solid state form, solid phase modification" of baricitinib and includes crystalline modifications, hydrates and solvates of baricitinib.

The term "crystal modification" of baricitinib, as used in this document, is synonymous to commonly used expressions "polymorphic form" or "crystalline form" of baricitinib.

The use of the term "about" includes and describes the value or parameter per se. For example, "about x" includes and describes "x" per se. In some embodiments, the term "about" when used in association with a measurement, or used to modify a value, a unit, a constant, or a range of values, refers to variations of +/- 20 percent, preferably +/- 10 percent and even more preferably +/- 5 percent.

The term "substantially" or "substantially free/pure" with respect to a particular solid form of a compound means that the polymorphic form contains about less than 30 percent, about less than 20 percent, about less than 15 percent, about less than 10 percent, about less than 5 percent, or about less than 1 percent by weight of impurities. In other embodiments, "substantially" or "substantially free/pure" refers to a substance free of impurities. Impurities may, for example, include by-products or left over reagents from chemical reactions, contaminants, degradation products, other polymorphic forms, water, and solvents.

It has now been surprisingly found that the above-mentioned crystalline salts of 2-[1-Ethylsulfonyl-3-[4-(7H-pyrrolo[2,3-d]pyrimidin-4-yl)pyrazol-1-yl]azetidin-3-yl]acetonitrile with at least one acid component HX, wherein HX is preferably is selected from the group consisting of hydrobromic acid, hydrochloric acid, sulfuric acid, methanesulfonic acid, ethanesulfonic acid, p-toluenesulfonic acid, L-tartaric acid, or fumaric acid can be prepared and have not been described in the literature yet and no solid state analytical data (X-Ray Powder Diffraction patterns, Single-Crystal X-Ray Diffraction data etc.) serving to characterize the crystalline phases have been provided.

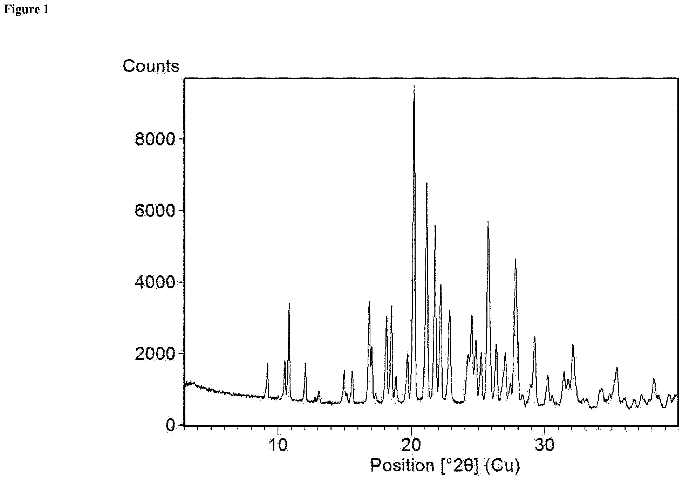

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrobromic acid salt according to the invention has the characteristic XRPD pattern as shown in **Figure 1****.** XRPD pattern was recorded on an X-Ray Powder Diffractometer (X'PERT PRO MPD PANalytical). The Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrobromic acid salt exhibits the following diffraction peaks in XRPD pattern, see **Table 1,** below:

**Table 1**

| **Pos. [°2θ]** | **d [Å]** | **Rel. Int. [%]** |
|---|---|---|
| 9.20 | 9.609 | 10.8 |
| 10.50 | 8.422 | 11.9 |
| 10.81 | 8.174 | 29.8 |
| 12.04 | 7.345 | 11.8 |
| 14.95 | 5.923 | 11.0 |
| 15.55 | 5.695 | 10.4 |
| 16.83 | 5.265 | 32.0 |
| 18.10 | 4.897 | 28.3 |
| 18.48 | 4.798 | 29.8 |
| 19.67 | 4.509 | 15.9 |
| 20.16 | 4.402 | 100.0 |
| 21.10 | 4.207 | 69.7 |
| 21.75 | 4.082 | 58.3 |
| 22.16 | 4.009 | 38.4 |
| 22.82 | 3.894 | 30.2 |
| 24.49 | 3.632 | 28.6 |
| 24.80 | 3.587 | 19.1 |
| 25.18 | 3.533 | 15.3 |
| 25.73 | 3.459 | 56.0 |
| 26.32 | 3.383 | 18.0 |
| 26.95 | 3.305 | 14.2 |
| 27.77 | 3.210 | 46.3 |
| 29.20 | 3.056 | 22.3 |
| 30.16 | 2.960 | 9.2 |
| 31.38 | 2.848 | 9.0 |
| 32.08 | 2.788 | 19.7 |
| 35.29 | 2.541 | 11.8 |
| 38.13 | 2.358 | 9.3 |

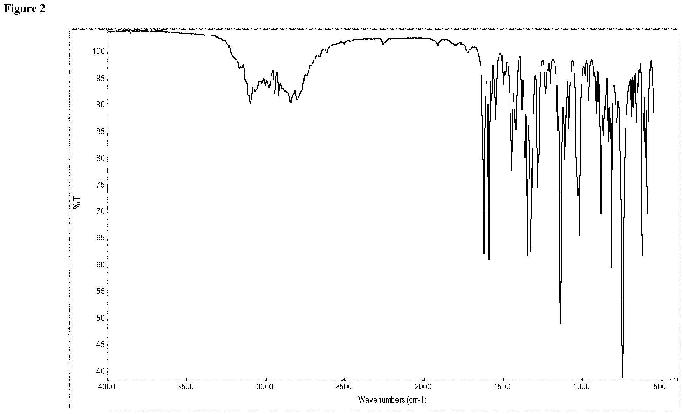

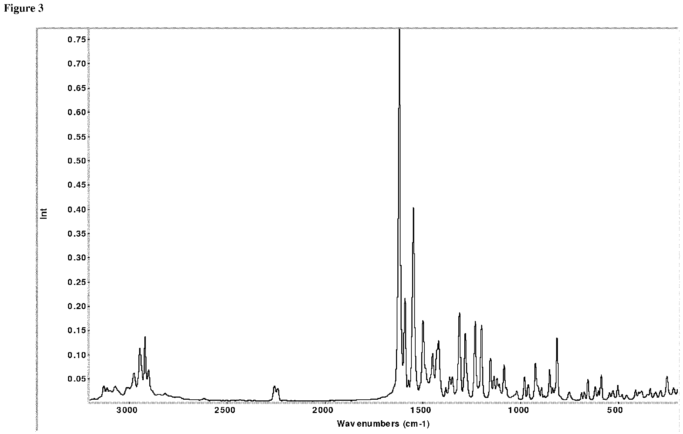

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrobromic acid salt can be characterized by FTIR and Raman spectroscopy. **Figure 2** shows the FTIR spectrum (Nicolet Thermo 6700c) comprising characteristic peaks at 3166, 3098, 2945, 2919, 2844, 2802, 1622, 1590, 1138 and 747 cm⁻¹ wavenumbers. The Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrobromic acid salt is characterised by a Raman spectrum (Bruker RFS 100/S) comprising characteristic peaks at 3193, 3130, 3072, 2882, 2823, 2392, 1286, 1137, 822 and 606 cm⁻¹ wavenumbers, shown in **Figure 3****.**

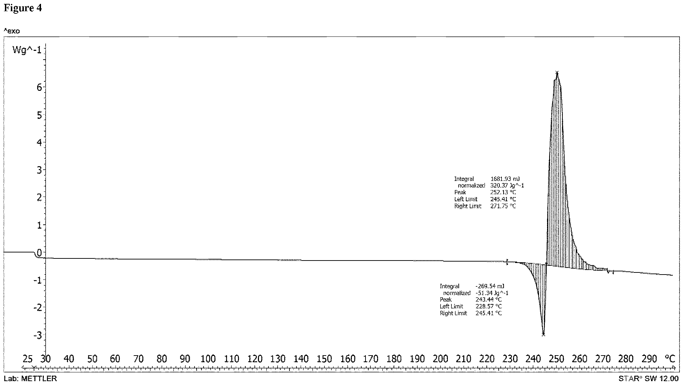

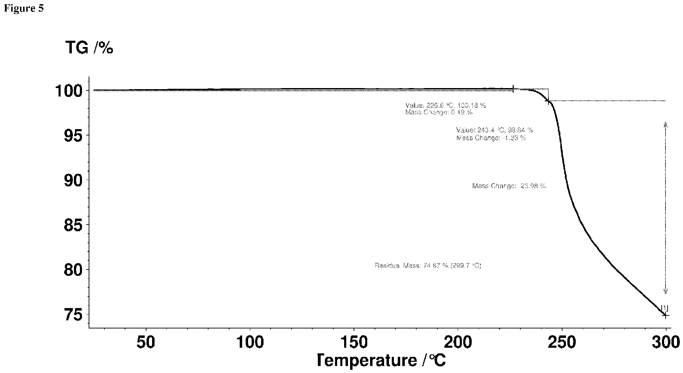

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrobromic acid salt can be further described by thermoanalytical methods (Differential Scanning Calorimetry, DSC; Thermal Gravimetric Analysis, TGA). **Figure 4** shows the DSC (Mettler-Toledo 822e DSC) and **Figure 5** shows the TGA (NETZSCH TG 209 thermogravimetric analyser) curves measured in the range of 25°C to 300°C and 25°C to 300°C, respectively. The Crystal modification 1 of 2-[1-Ethylsulfonyl-3-[4-(7H-pyrrolo[2,3-d]pyrimidin-4-yl)pyrazol-1-yllazetidin-3-yl]acetonitrile hydrobromic acid salt shows a 0.2% weigh loss in the range of 25°C to 226°C. The DSC measurement gives a melting process with T_{onset,1}=228.6°C; and Tₚₑₐₖ=243.4°C.

In one of the aspects of the invention, an alternative process for preparation of the Crystal modification 1 is provided. In this process, baricitinib free base is dissolved in a suitable organic solvent by heating of the system to a temperature 50°C. The counterion is added to the solution of baricitinib and then kept at the temperature of 50°C for additional 1 hour. The suspension obtained is then cooled back to room temperature. After filtering off and drying at laboratory conditions, the product was analysed by the methods described above and characterised as the Crystal modification 1 of baricitinib hydrobromic acid salt.

The suitable organic solvent is selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably tetrahydrofuran.

The process of preparation of the Crystal modification 1 of baricitinib hydrobromic acid salt thus comprises the steps of:
a/ dissolution of baricitinib freebase in a suitable organic solvent at the temperature of 50°C;
b/ drop-wise addition of the hydrobromic acid 48% aqueous solution;
c/ cooling the solution of the step b/ to a temperature of 0-25°C while precipitation occurs;
d/ keeping the suspension of the step c/ for about 16 hours at a temperature of 0-25°C;
e/ isolating the baricitinib hydrobromic acid salt in Crystal modification 1
f/ optionally, drying of the product of step e/ at laboratory condition until the constant weight of the product is reached.

The suitable organic solvent is selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably tetrahydrofuran, even more preferably the suitable organic solvent is tetrahydrofuran at the temperature of 50°C.

Another process for the preparation of the Crystal modification 1 of baricitinib hydrobromic acid salt comprises the steps of:
a/ suspending baricitinib hydrobromic acid salt in 2-propanol at room temperature;
b/ stirring the suspension of step a/ at 50°C for 72 hours;
c/ isolating the Crystal modification 1 of baricitinib hydrobromic acid salt;
d/ optionally, drying of the product of step c/ at laboratory condition until the constant weight of the product is reached.

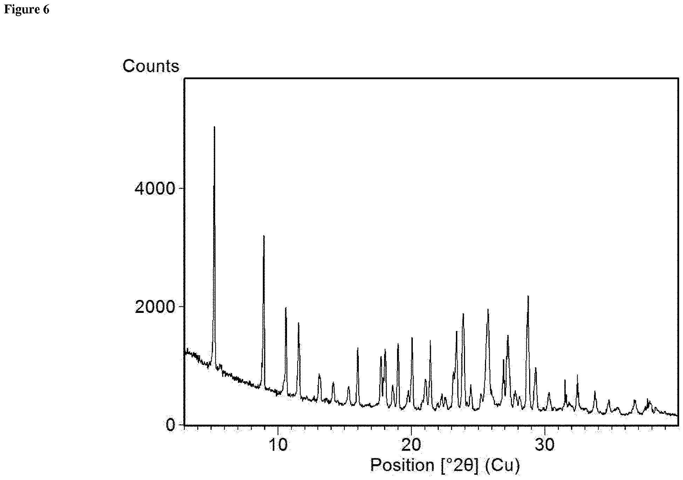

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt according to the invention has the characteristic XRPD pattern as shown in **Figure 6****.** XRPD pattern was recorded on an X-Ray Powder Diffractometer (X'PERT PRO MPD PANalytical). The Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt exhibits the following diffraction peaks in XRPD pattern, see **Table 2,** below:

**Table 2**

| **Pos. [°2θ]** | **d [Å]** | **Rel. Int. [%]** |
|---|---|---|
| 5.25 | 16.835 | 100.0 |
| 8.92 | 9.906 | 65.4 |
| 10.59 | 8.351 | 41.1 |
| 11.53 | 7.668 | 33.1 |
| 13.07 | 6.769 | 11.7 |
| 14.13 | 6.261 | 9.4 |
| 15.23 | 5.814 | 6.5 |
| 15.97 | 5.545 | 25.7 |
| 17.70 | 5.007 | 23.4 |
| 18.02 | 4.920 | 25.9 |
| 18.59 | 4.769 | 9.9 |
| 18.98 | 4.672 | 28.0 |
| 20.02 | 4.431 | 30.0 |
| 21.02 | 4.223 | 12.6 |
| 21.40 | 4.149 | 31.8 |
| 23.35 | 3.807 | 32.3 |
| 23.85 | 3.727 | 43.7 |
| 24.43 | 3.641 | 9.3 |
| 25.72 | 3.461 | 35.9 |
| 26.85 | 3.318 | 16.6 |
| 27.23 | 3.272 | 33.7 |
| 28.72 | 3.106 | 49.0 |
| 29.26 | 3.049 | 17.5 |
| 30.29 | 2.949 | 7.6 |
| 31.51 | 2.837 | 17.8 |
| 32.42 | 2.759 | 17.3 |
| 33.72 | 2.656 | 10.4 |
| 34.74 | 2.580 | 5.6 |
| 36.69 | 2.447 | 7.5 |

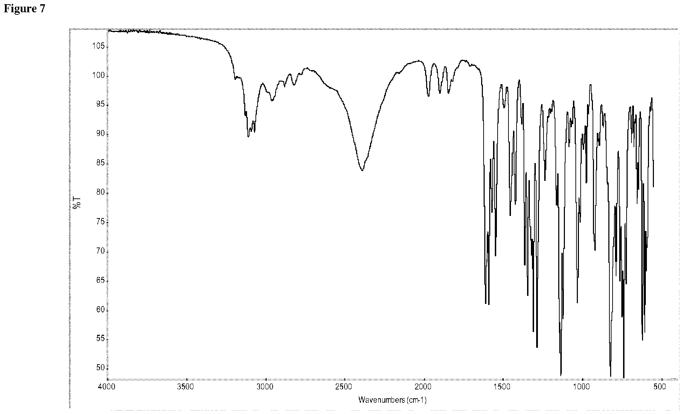

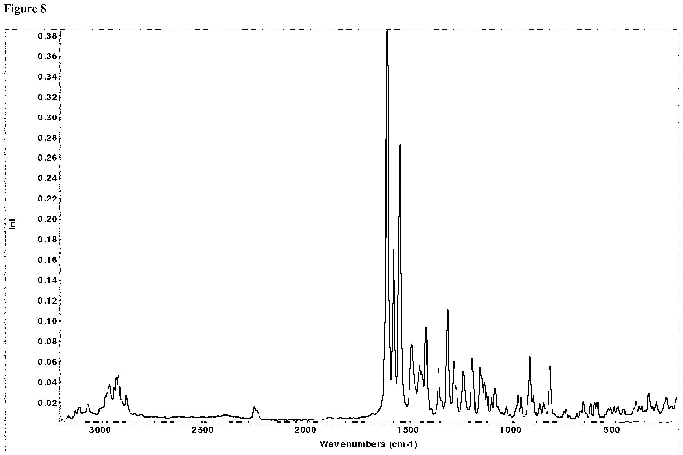

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt can be characterized by FTIR and Raman spectroscopy. **Figure 7** shows the FTIR spectrum (Nicolet Thermo 6700c) comprising characteristic peaks at 3193, 3130, 3072, 2882, 2823, 2392, 1286, 1137, 822 and 606 cm⁻¹ wavenumbers. The Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt is characterised by a Raman spectrum (Bruker RFS 100/S) comprising characteristic peaks at 3132, 3114, 3073, 2966, 2922, 2885, 2260, 1614, 1581, and 1552 cm⁻¹ wavenumbers, shown in **Figure 8****.**

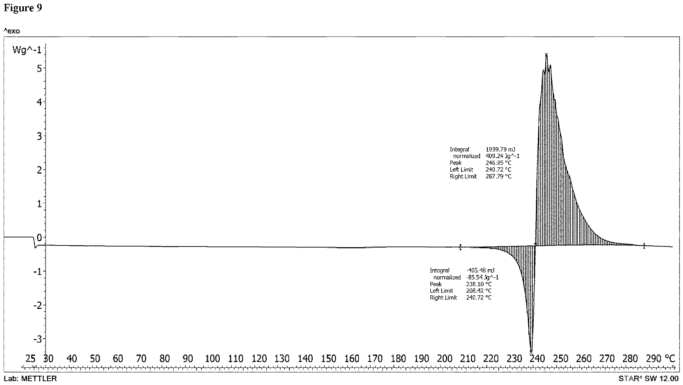

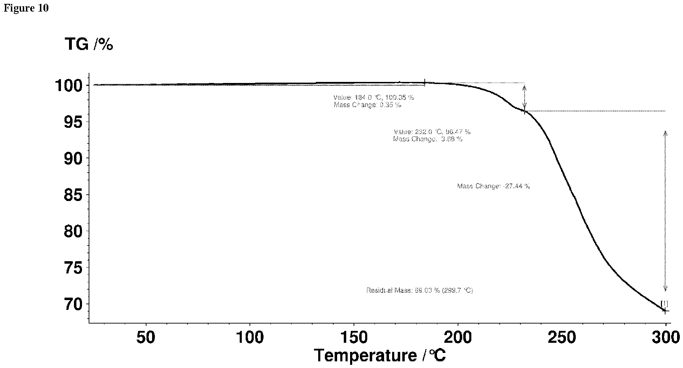

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt can be further described by thermal analytical methods. **Figure 9** shows the DSC (Mettler-Toledo 822e DSC) and **Figure 10** shows the TGA (NETZSCH TG 209 thermogravimetric analyser) curves measured in the range of 25°C to 300°C and 25°C to 300°C, respectively. The Crystal modification 2 of 2-[1-Ethylsulfonyl-3-[4-(7H-pyrrolo[2,3-d]pyrimidin-4-yl)pyrazol-1-yl]azetidin-3-yl]acetonitrile hydrobromic acid salt shows a 0.35% weigh loss in the range of 25°C to 184°C. The DSC measurement gives a melting process with T_{onset,1}=208.4°C; and Tₚₑₐₖ=238.1°C.

The Crystal modification 1 of baricitinib hydrochloric acid salt can be prepared by a process comprising the steps of:
a/ dissolution of baricitinib free in a suitable organic solvent at the temperature of 50°C;
b/ drop-wise addition of the hydrochloric acid 35% aqueous solution;
c/ cooling the solution of the step b/ to a temperature of 0-25°C while precipitation occurs;
d/ keeping the suspension of the step c/ for about 16 hours at a temperature of 0-25°C;
e/ isolating the baricitinib hydrochloric acid salt in Crystal modification 1
f/ optionally, drying of the product of step e/ at laboratory condition until the constant weight of the product is reached.

The suitable organic solvent is selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably tetrahydrofuran, even more preferably the suitable organic solvent is tetrahydrofuran at the temperature of 50°C.

Another process for the preparation of the Crystal modification 1 of baricitinib hydrochloric acid salt comprises the steps of:
a/ suspending baricitinib hydrochloric acid salt in acetone at room temperature;
b/ stirring the suspension of step a/ at 50°C for 72 hours;
c/ isolating the Crystal modification 1 of baricitinib hydrochloric acid salt;
d/ optionally, drying of the product of step c/ at laboratory condition until the constant weight of the product is reached.

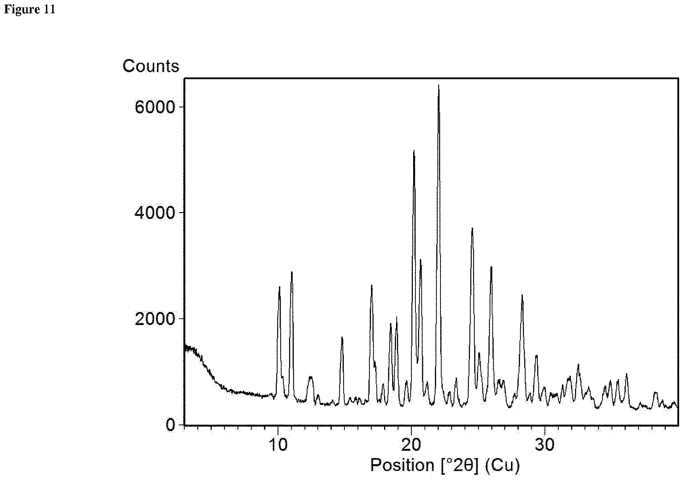

The Crystal modification 2 of [1-Ethylsulfonyl-3-[4-(7H-pyrrolo[2,3-d]pyrimidin-4-yl)pyrazol-1-yl]azetidin-3-yl]acetonitrile hydrochloric acid salt according to the invention has the characteristic XRPD pattern as shown in **Figure** 11. XRPD pattern was recorded on an X-Ray Powder Diffractometer (X'PERT PRO MPD PANalytical). The Crystal modification 2 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt exhibits the following diffraction peaks in XRPD pattern, see **Table 3,** below:

**Table 3**

| **Pos. [°2θ]** | **d [Å]** | **Rel. Int. [%]** |
|---|---|---|
| 10.12 | 8.734 | 33.4 |
| 11.06 | 7.995 | 36.6 |
| 12.34 | 7.165 | 7.5 |
| 14.80 | 5.980 | 20.2 |
| 17.00 | 5.212 | 37.4 |
| 17.85 | 4.964 | 6.3 |
| 18.46 | 4.803 | 22.5 |
| 18.90 | 4.692 | 23.0 |
| 19.59 | 4.527 | 7.0 |
| 20.20 | 4.393 | 74.0 |
| 20.66 | 4.297 | 42.4 |
| 22.01 | 4.036 | 100.0 |
| 23.32 | 3.811 | 8.0 |
| 24.52 | 3.628 | 54.0 |
| 25.07 | 3.550 | 14.9 |
| 25.93 | 3.433 | 43.0 |
| 26.52 | 3.358 | 6.9 |
| 26.87 | 3.316 | 7.2 |
| 28.25 | 3.157 | 33.1 |
| 29.32 | 3.043 | 14.8 |
| 29.87 | 2.989 | 5.8 |
| 31.29 | 2.857 | 6.0 |
| 31.87 | 2.805 | 7.6 |
| 32.48 | 2.755 | 12.1 |
| 33.13 | 2.702 | 5.7 |
| 34.45 | 2.602 | 6.2 |
| 34.86 | 2.571 | 8.0 |
| 35.40 | 2.533 | 8.9 |
| 36.08 | 2.487 | 11.7 |

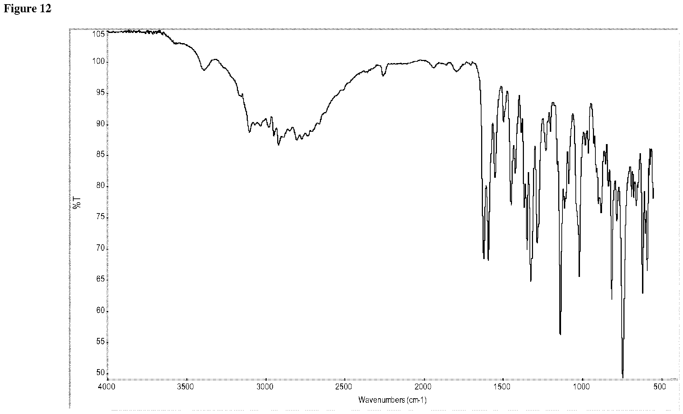

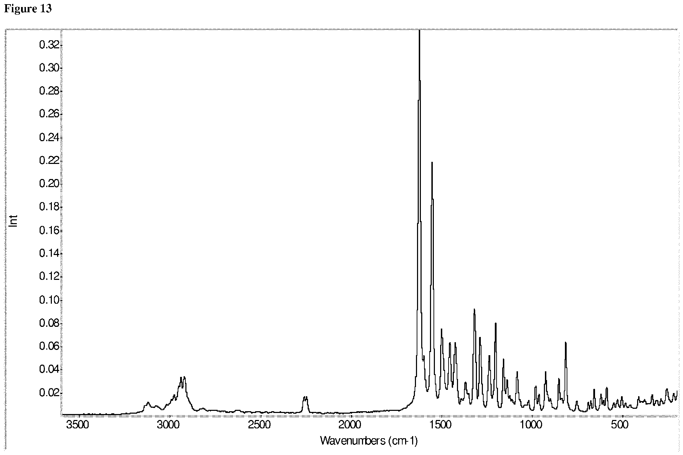

The Crystal modification 2 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt can be characterized by FTIR and Raman spectroscopy. **Figure 12** shows the FTIR spectrum (Nicolet Thermo 6700c) comprising characteristic peaks at 3391, 3104, 2982, 2950, 2920, 2804, 2259, 1623, 1139 and 745 cm⁻¹ wavenumbers. The Crystal modification 2 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt is characterised by a Raman spectrum (Bruker RFS 100/S) comprising characteristic peaks at 3123, 2979, 2940, 2923, 2246, 1624, 1553, 1320, 1203 and 816 cm⁻¹ wavenumbers, shown in **Figure 13****.**

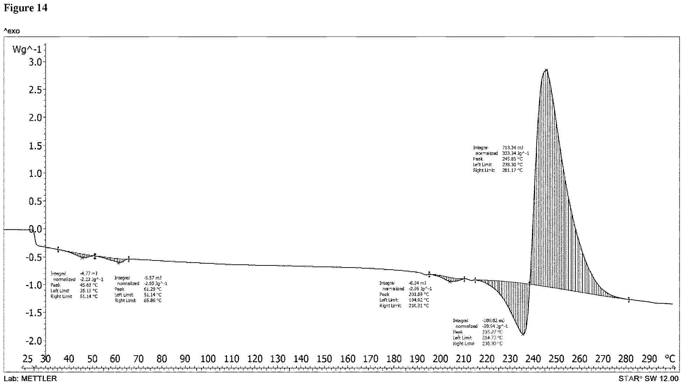

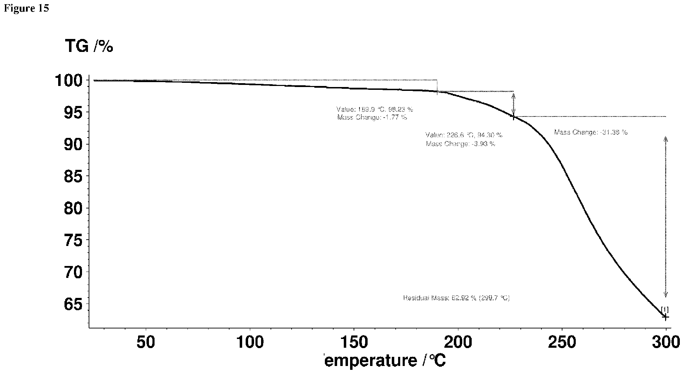

The Crystal modification 2 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt can be further described by thermal analytical methods. **Figure 14** shows the DSC (Mettler-Toledo 822e DSC) and **Figure 15** shows the TGA (NETZSCH TG 209 thermogravimetric analyser) curves measured in the range of 25°C to 300°C and 25°C to 300°C, respectively. The Crystal modification 2 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt shows a 1.8% weigh loss in the range of 25°C to 190°C. The DSC measurement gives a melting process with Tₒₙₛₑₜ=214.73°C and Tₚₑₐₖ=235.27°C.

The Crystal modification 2 of baricitinib hydrochloric acid salt can be prepared by a process comprising the steps of:
a/ suspending baricitinib hydrochloric acid salt in water at 50°C;
b/ stirring the suspension of step a/ at room temperature for 72 hours;
c/ isolating the Crystal modification 2 of baricitinib hydrochloric acid salt;
d/ optionally, drying of the product of step c/ at laboratory condition until the constant weight of the product is reached.

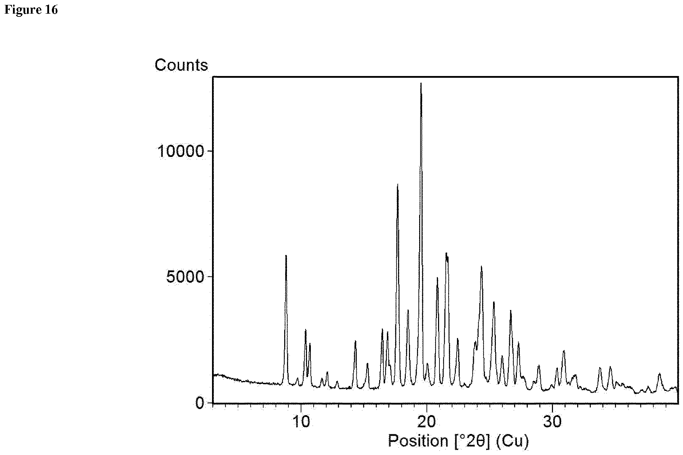

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile sulfuric acid salt according to the invention has the characteristic XRPD pattern as shown in **Figure 16****.** XRPD pattern was recorded on an X-Ray Powder Diffractometer (X'PERT PRO MPD PANalytical). The Crystal modification 1 of 2-[1-Ethylsulfonyl-3-[4-(7H-pyrrolo[2,3-d]pyrimidin-4-yl)pyrazol-1-yl]azetidin-3-yl]acetonitrile sulfuric acid salt exhibits the following diffraction peaks in XRPD pattern, see **Table 4,** below:

**Table 4**

| Pos. [°2θ] | d [Å] | Rel. Int. [%] |
|---|---|---|
| 8.80 | 10.045 | 41.5 |
| 10.34 | 8.545 | 18.9 |
| 10.68 | 8.281 | 13.8 |
| 12.07 | 7.329 | 5.5 |
| 14.29 | 6.195 | 16.6 |
| 15.25 | 5.805 | 8.8 |
| 16.42 | 5.393 | 19.5 |
| 16.86 | 5.254 | 18.0 |
| 17.66 | 5.018 | 65.6 |
| 18.49 | 4.795 | 25.9 |
| 19.51 | 4.547 | 100.0 |
| 20.81 | 4.265 | 36.7 |
| 21.52 | 4.126 | 41.1 |
| 22.42 | 3.963 | 16.1 |
| 24.33 | 3.656 | 40.2 |
| 25.28 | 3.520 | 27.7 |
| 25.97 | 3.428 | 9.0 |
| 26.67 | 3.340 | 25.0 |
| 27.26 | 3.269 | 15.3 |
| 28.87 | 3.090 | 8.7 |
| 30.32 | 2.946 | 7.9 |
| 30.90 | 2.892 | 11.7 |
| 33.74 | 2.654 | 8.8 |
| 34.56 | 2.593 | 9.1 |
| 38.48 | 2.338 | 6.0 |

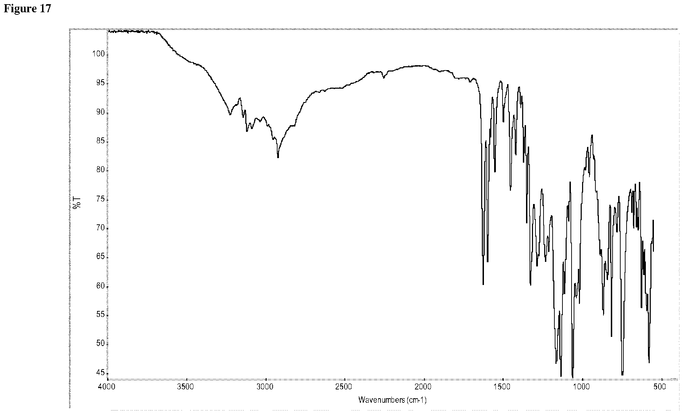

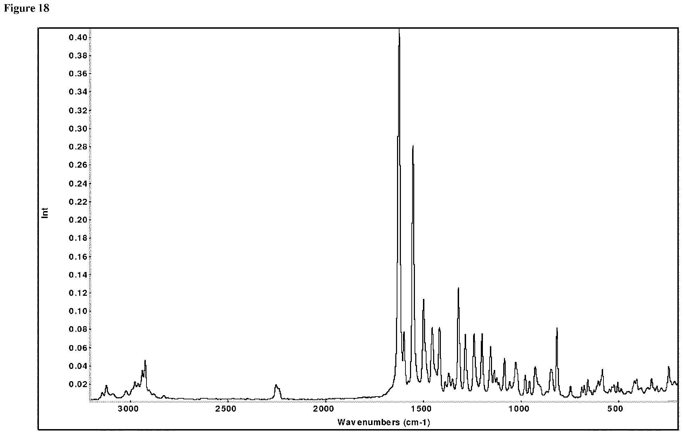

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile sulfuric acid salt can be characterized by FTIR and Raman spectroscopy. **Figure 17** shows the FTIR spectrum (Nicolet Thermo 6700c) comprising characteristic peaks at 3226, 3119, 2925, 2254, 1626, 1327, 1135, 1061, 744 and 579 cm⁻¹ wavenumbers. The Crystal modification 1 of 2-[1-Ethylsulfonyl-3-[4-(7H-pyrrolo[2,3-d]pyrimidin-4-yl)pyrazol-1-yl]azetidin-3-yl]acetonitrile sulfuric acid salt is characterised by a Raman spectrum (Bruker RFS 100/S) comprising characteristic peaks at 3145, 3124, 2940, 2927, 2256, 1626, 1554, 1500, 1321 and 817 cm⁻¹ wavenumbers, shown in **Figure 18****.**

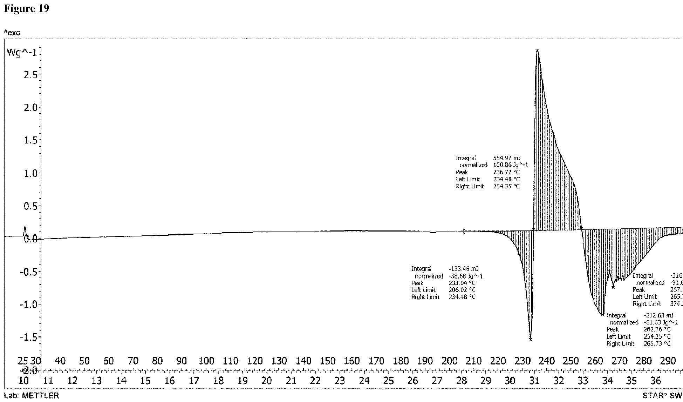

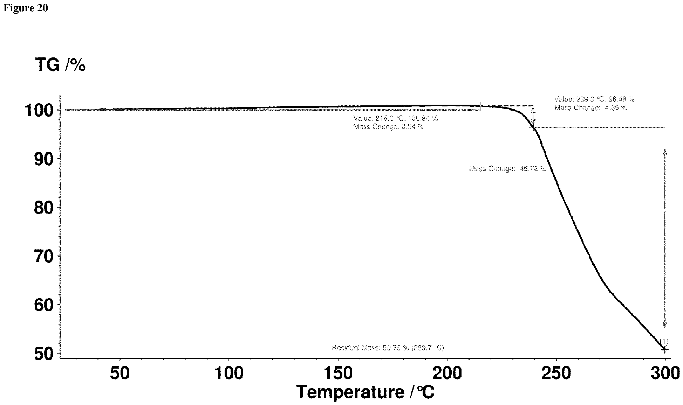

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile sulfuric acid salt can be further described by thermal analytical methods. **Figure 19** shows the DSC (Mettler-Toledo 822e DSC) and **Figure 20** shows the TGA (NETZSCH TG 209 thermogravimetric analyser) curves measured in the range of 25°C to 300°C and 25°C to 300°C, respectively. The Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrobromic acid salt shows a 0.8% weigh loss in the range of 25°C to 215°C. The DSC measurement gives a melting process with T_{onset,1}=206.0°C and Tₚₑₐₖ=236.0°C.

The Crystal modification 1 of baricitinib sulfuric acid salt can be prepared by a process comprising the steps of:
a/ dissolution of baricitinib free in a suitable organic solvent at the temperature of 50°C;
b/ drop-wise addition of the sulfuric acid 96% aqueous solution;
c/ cooling the solution of the step b/ to a temperature of 0-25°C while precipitation occurs;
d/ keeping the suspension of the step c/ for about 16 hours at a temperature of 0-25°C;
e/ isolating the baricitinib sulfuric acid salt in Crystal modification 1
f/ optionally, drying of the product of step e/ at laboratory condition until the constant weight of the product is reached.

The suitable organic solvent is selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably tetrahydrofuran, even more preferably the suitable organic solvent is tetrahydrofuran at the temperature of 50°C.

Another process for the preparation of the Crystal modification 1 of baricitinib sulfuric acid salt comprises the steps of:
a/ suspending baricitinib sulfuric acid salt in ethyl acetate at room temperature;
b/ stirring the suspension of step a/ at 50°C for 72 hours;
c/ isolating the Crystal modification 1 of baricitinib sulfuric acid salt;
d/ optionally, drying of the product of step c/ at laboratory condition until the constant weight of the product is reached.

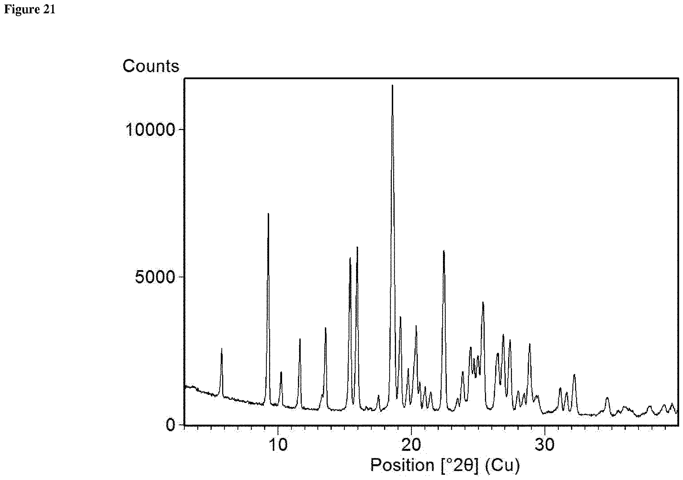

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile methanesulfonic acid salt according to the invention has the characteristic XRPD pattern as shown in **Figure 21****.** XRPD pattern was recorded on an X-Ray Powder Diffractometer (X'PERT PRO MPD PANalytical). The Crystal modification 1 of 2-[1-Ethylsulfonyl-3-[4-(7H-pyrrolo[2,3-d]pyrimidin-4-yl)pyrazol-1-yl]azetidin-3-yl]acetonitrile methanesulfonic acid salt exhibits the following diffraction peaks in XRPD pattern, see **Table 5,** below:

**Table 5**

| Pos. [°2θ] | d [Å] | Rel. Int. [%] |
|---|---|---|
| 5.77 | 15.311 | 15.0 |
| 9.26 | 9.548 | 58.8 |
| 10.22 | 8.645 | 11.7 |
| 11.62 | 7.611 | 22.6 |
| 13.55 | 6.531 | 26.5 |
| 15.37 | 5.759 | 48.4 |
| 15.91 | 5.567 | 50.4 |
| 18.55 | 4.779 | 100.0 |
| 19.14 | 4.634 | 29.7 |
| 19.72 | 4.499 | 10.7 |
| 20.29 | 4.374 | 24.7 |
| 20.99 | 4.229 | 7.3 |
| 21.42 | 4.145 | 6.7 |
| 22.40 | 3.965 | 52.5 |
| 23.81 | 3.734 | 12.6 |
| 24.40 | 3.646 | 21.1 |
| 24.95 | 3.566 | 17.3 |
| 25.37 | 3.508 | 34.1 |
| 26.47 | 3.364 | 14.0 |
| 26.84 | 3.319 | 24.9 |
| 27.34 | 3.259 | 22.6 |
| 27.95 | 3.190 | 6.6 |
| 28.82 | 3.096 | 22.9 |
| 31.11 | 2.872 | 8.7 |
| 31.58 | 2.831 | 6.9 |
| 32.16 | 2.781 | 13.1 |
| 34.67 | 2.585 | 3.9 |

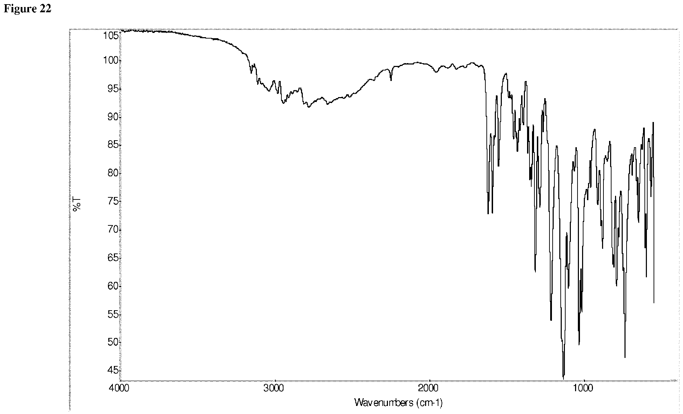

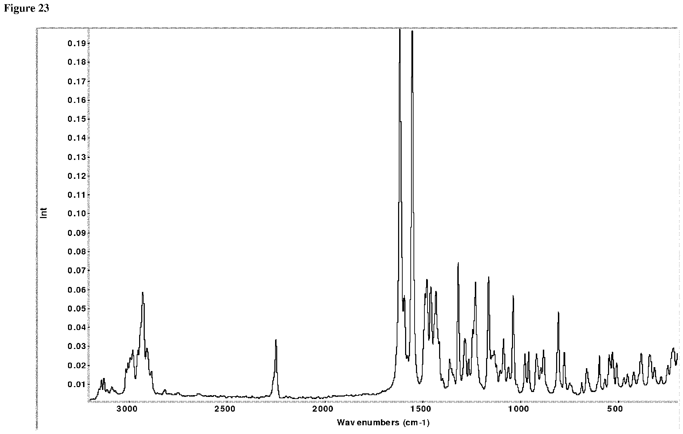

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile methanesulfonic acid salt can be characterized by FTIR and Raman spectroscopy. **Figure 22** shows the FTIR spectrum (Nicolet Thermo 6700c) comprising characteristic peaks at 3154, 3114, 2983, 2251, 1556, 1318, 1217, 1136, 1035 and 739 cm⁻¹ wavenumbers. The Crystal modification 1 of 2-[1-Ethylsulfonyl-3-[4-(7H-pyrrolo[2,3-d]pyrimidin-4-yl)pyrazol-1-yl]azetidin-3-yl]acetonitrile methanesulfonic acid salt is characterised by a Raman spectrum (Bruker RFS 100/S) comprising characteristic peaks at 3144, 3131, 2983, 2934, 2252, 1619, 1556, 1321, 1165 and 809 cm⁻¹ wavenumbers, shown in **Figure 23****.**

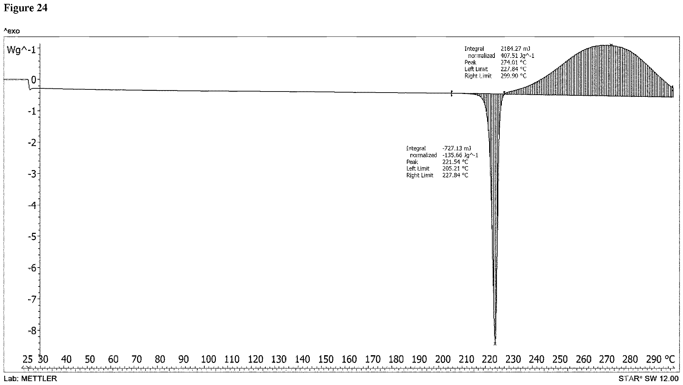

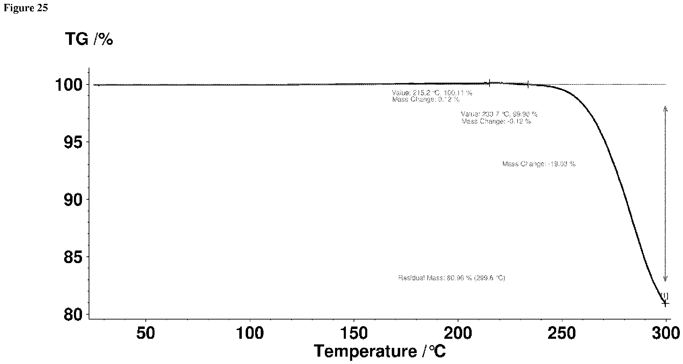

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile methanesulfonic acid salt can be further described by thermal analytical methods. **Figure 24** shows the DSC (Mettler-Toledo 822e DSC) and **Figure 25** shows the TGA (NETZSCH TG 209 thermogravimetric analyser) curves measured in the range of 25°C to 300°C and 25°C to 300°C, respectively. The Crystal modification 1 of 2-[1-Ethylsulfonyl-3-[4-(7H-pyrrolo[2,3-d]pyrimidin-4-yl)pyrazol-1-yl]azetidin-3-yl]acetonitrile methanesulfonic acid salt shows a 0.12% weigh loss in the range of 25°C to 215°C. The DSC measurement gives a melting process with T_{onset,1}=205.21°C; and Tₚₑₐₖ=221.5°C.

The Crystal modification 1 of baricitinib methanesulfonic acid salt can be prepared by a process comprising the steps of:
a/ dissolution of baricitinib free base in a suitable organic solvent;
b/ drop-wise addition of the methanesulfonic acid >98% aqueous solution;
c/ stirring the solution of the step b/ at 50°C for additional 1 hour;
d/ cooling the solution of the step c/ to room temperature;
e/ keeping the solution of the step d/ for 16 hours at room temperature while precipitation occurred;
f/ isolating the baricitinib methanesulfonic acid salt in Crystal modification 1;
g/ optionally, drying of the product of step f/ at laboratory condition until the constant weight of the product is reached.

The suitable organic solvent is selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably tetrahydrofuran, even more preferably the suitable organic solvent is tetrahydrofuran at the temperature of 50°C.

Another process for the preparation of the Crystal modification 1 of baricitinib methanesulfonic acid salt comprises the steps of:
a/ suspending baricitinib methanesulfonic acid salt in methanol-water 1-1 V/V mixture at 50°C;
b/ stirring the suspension of step a/ at 50°C for 72 hours;
c/ isolating the Crystal modification 1 of baricitinib methanesulfonic acid salt;
d/ optionally, drying of the product of step c/ at laboratory condition until the constant weight of the product is reached.

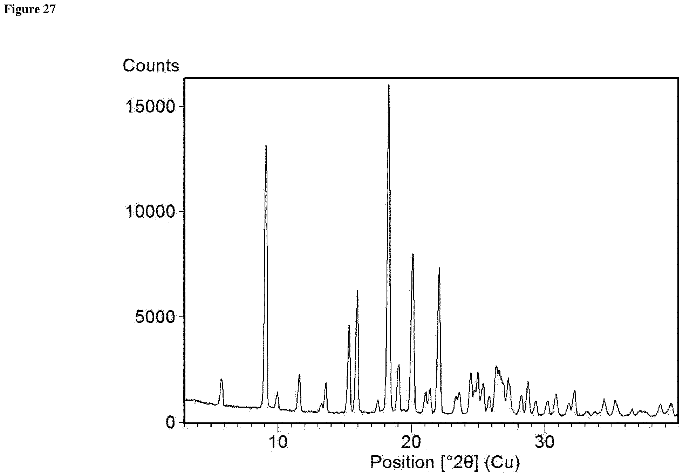

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile ethanesulfonic acid salt according to the invention has the characteristic XRPD pattern as shown in **Figure 27****.** XRPD pattern was recorded on an X-Ray Powder Diffractometer (X'PERT PRO MPD PANalytical). The Crystal modification 1 of 2-[1-Ethylsulfonyl-3-[4-(7H-pyrrolo[2,3-d]pyrimidin-4-yl)pyrazol-1-yl]azetidin-3-yl]acetonitrile ethanesulfonic acid salt exhibits the following diffraction peaks in XRPD pattern, see **Table 6,** below:

**Table 6**

| **Pos. [°2θ]** | **d [Å]** | **Rel. Int. [%]** |
|---|---|---|
| 5.73 | 15.401 | 7.6 |
| 9.08 | 9.737 | 80.6 |
| 9.92 | 8.908 | 5.5 |
| 11.57 | 7.644 | 11.2 |
| 13.56 | 6.527 | 9.1 |
| 15.29 | 5.790 | 26.8 |
| 15.96 | 5.549 | 30.1 |
| 18.26 | 4.855 | 100.0 |
| 19.00 | 4.668 | 14.0 |
| 20.05 | 4.425 | 50.0 |
| 21.03 | 4.220 | 6.1 |
| 21.36 | 4.157 | 8.2 |
| 22.02 | 4.033 | 46.2 |
| 23.54 | 3.776 | 5.9 |
| 24.42 | 3.643 | 12.3 |
| 24.94 | 3.567 | 13.8 |
| 25.38 | 3.506 | 9.4 |
| 25.79 | 3.451 | 5.1 |
| 26.31 | 3.384 | 11.5 |
| 27.26 | 3.269 | 10.1 |
| 28.19 | 3.163 | 5.7 |
| 28.70 | 3.108 | 10.7 |
| 29.27 | 3.049 | 4.2 |
| 30.15 | 2.962 | 4.5 |
| 30.78 | 2.902 | 6.8 |
| 32.16 | 2.781 | 8.3 |
| 34.39 | 2.606 | 4.9 |
| 35.28 | 2.542 | 5.1 |
| 38.59 | 2.331 | 4.0 |

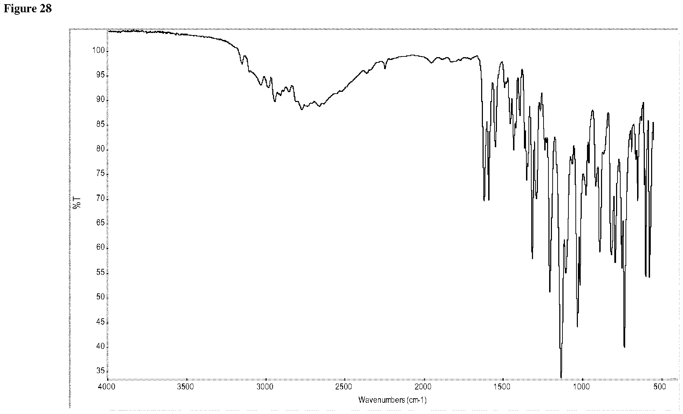

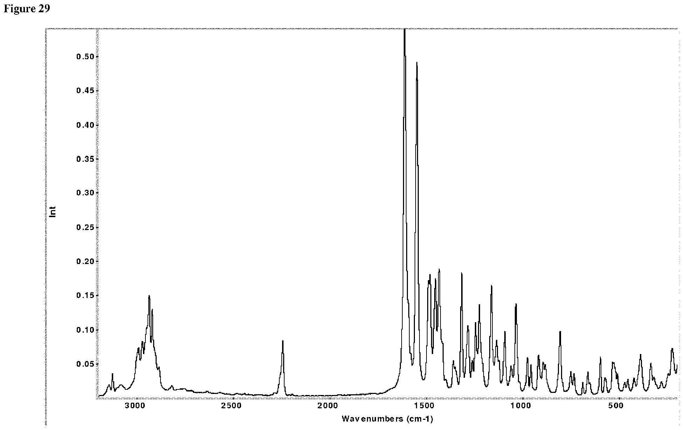

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile ethanesulfonic acid salt can be characterized by FTIR and Raman spectroscopy. **Figure 28** shows the FTIR spectrum (Nicolet Thermo 6700c) comprising characteristic peaks at 3151, 2943, 2773, 2246, 1620, 1206, 1134, 1031, 734 and 576 cm⁻¹ wavenumbers. The Crystal modification 1 of 2-[1-Ethylsulfonyl-3-[4-(7H-pyrrolo[2,3-d]pyrimidin-4-yl)pyrazol-1-yl]azetidin-3-yl]acetonitrile ethanesulfonic acid salt is characterised by a Raman spectrum (Bruker RFS 100/S) comprising characteristic peaks at 3145, 3128, 2939, 2923, 2246, 1613, 1551, 1483, 1165 and 808 cm⁻¹ wavenumbers, shown in **Figure 29****.**

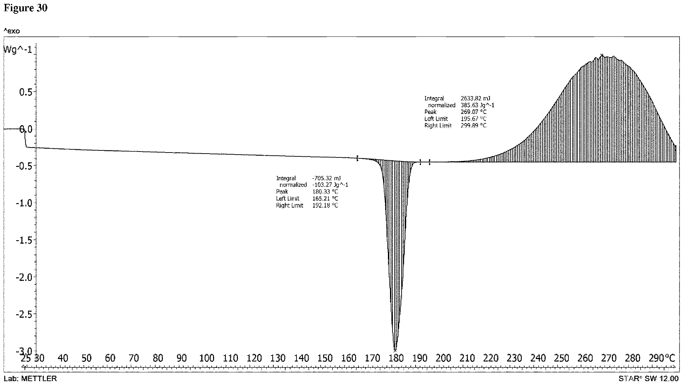

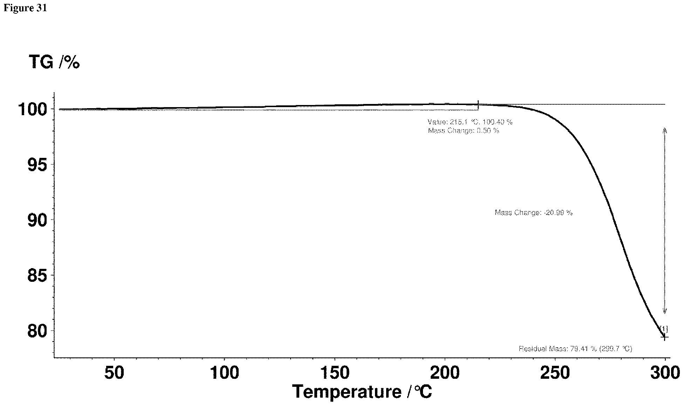

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile ethanesulfonic acid salt can be further described by thermal analytical methods. **Figure 30** shows the DSC (Mettler-Toledo 822e DSC) and **Figure 31** shows the TGA (NETZSCH TG 209 thermogravimetric analyser) curves measured in the range of 25°C to 300°C and 25°C to 300°C, respectively. The Crystal modification 1 of 2-[1-Ethylsulfonyl-3-[4-(7H-pyrrolo[2,3-d]pyrimidin-4-yl)pyrazol-1-yl]azetidin-3-yl]acetonitrile ethanesulfonic acid salt shows a 0.5% weigh loss in the range of 25°C to 215°C. The DSC measurement gives a melting process with T_{onset,1}=165.1°C; and Tₚₑₐₖ=180.3°C.

The Crystal modification 1 of baricitinib ethanesulfonic acid salt can be prepared by a process comprising the steps of:
a/ dissolution of baricitinib free base in a suitable organic solvent;
b/ drop-wise addition of the ethanesulfonic acid 70% aqueous solution;
c/ stirring the solution of the step b/ at 50°C for additional 1 hour;
d/ cooling the solution of the step c/ to room temperature;
e/ keeping the solution of the step d/ for 16 hours at room temperature while precipitation occurred;
f/ isolating the baricitinib ethanesulfonic acid salt in Crystal modification 1;
g/ optionally, drying of the product of step f/ at laboratory condition until the constant weight of the product is reached.

The suitable organic solvent is selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably tetrahydrofuran, even more preferably the suitable organic solvent is tetrahydrofuran at the temperature of 50°C.

Another process for the preparation of the Crystal modification 1 of baricitinib ethanesulfonic acid salt comprises the steps of:
a/ suspending baricitinib ethanesulfonic acid salt in ethyl acetate at 50°C;
b/ stirring the suspension of step a/ at 50°C for 72 hours;
c/ isolating the Crystal modification 1 of baricitinib ethanesulfonic acid salt;
d/ optionally, drying of the product of step c/ at laboratory condition until the constant weight of the product is reached.

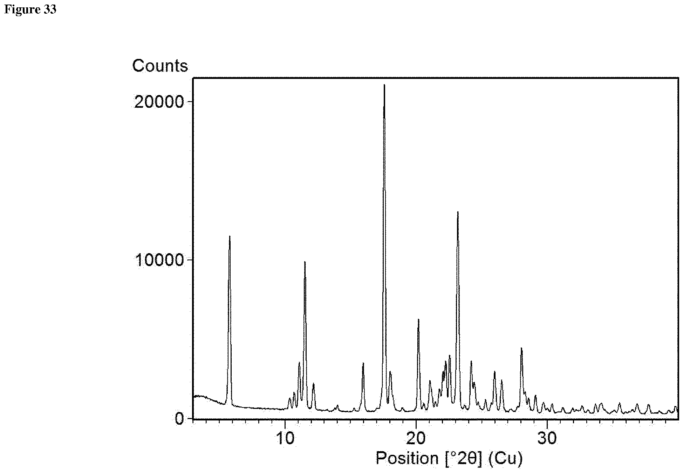

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt according to the invention has the characteristic XRPD pattern as shown in **Figure 33****.** XRPD pattern was recorded on an X-Ray Powder Diffractometer (X'PERT PRO MPD PANalytical). The Crystal modification 1 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt exhibits the following diffraction peaks in XRPD pattern, see **Table 7,** below:

**Table 7**

| **Pos. [°2θ]** | **d [Å]** | **Rel. Int. [%]** |
|---|---|---|
| 5.76 | 15.327 | 48.1 |
| 11.08 | 7.977 | 13.4 |
| 11.50 | 7.686 | 41.1 |
| 12.17 | 7.269 | 7.3 |
| 15.95 | 5.554 | 14.5 |
| 17.56 | 5.048 | 100.0 |
| 18.01 | 4.920 | 10.5 |
| 20.16 | 4.401 | 26.8 |
| 21.07 | 4.213 | 8.7 |
| 22.02 | 4.033 | 11.3 |
| 22.23 | 3.996 | 13.5 |
| 22.54 | 3.942 | 17.1 |
| 23.15 | 3.840 | 58.8 |
| 24.17 | 3.679 | 14.8 |
| 25.96 | 3.430 | 11.9 |
| 26.51 | 3.360 | 9.0 |
| 28.01 | 3.183 | 19.9 |
| 29.07 | 3.069 | 5.5 |
| 29.67 | 3.008 | 3.1 |

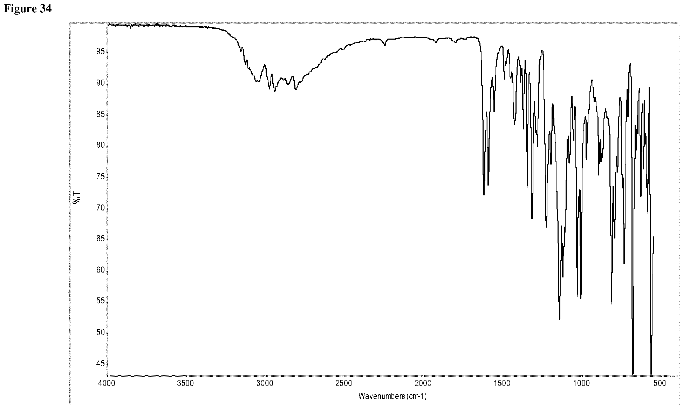

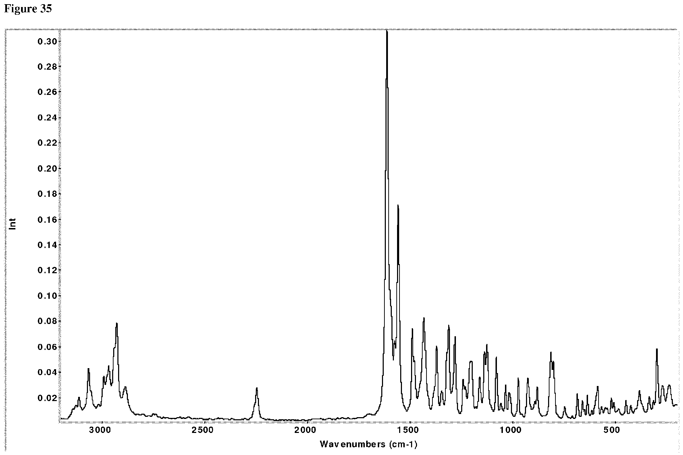

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt can be characterized by FTIR and Raman spectroscopy. **Figure 34** shows the FTIR spectrum (Nicolet Thermo 6700c) comprising characteristic peaks at 3159, 2978, 2811, 2248, 1317, 1227, 1145, 814, 682 and 564cm⁻¹ wavenumbers. The Crystal modification 1 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt is characterised by a Raman spectrum (Bruker RFS 100/S) comprising characteristic peaks at 3115, 3068, 2932, 2890, 2249, 1615, 1560, 1435, 1314, and 815 cm⁻¹ wavenumbers, shown in **Figure 35****.**

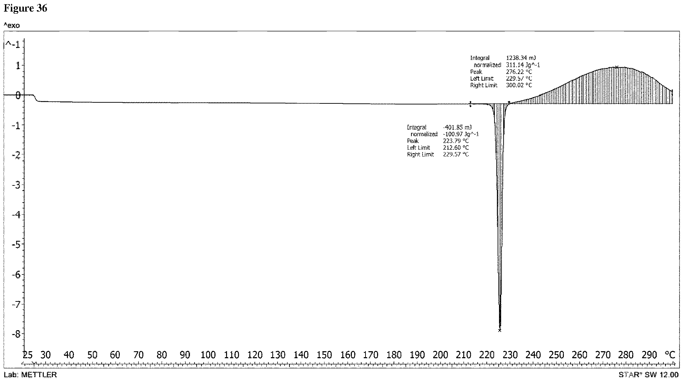

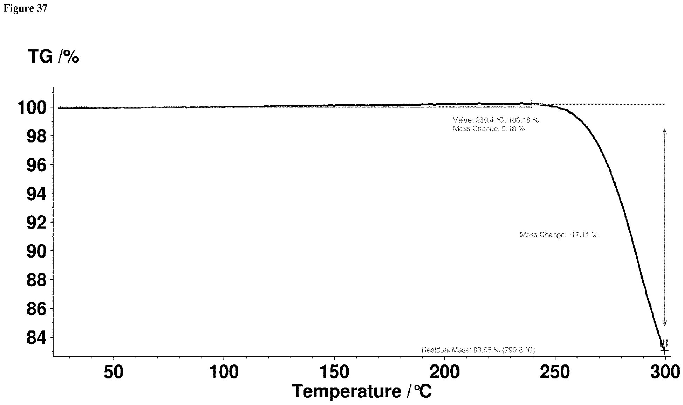

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt can be further described by thermoanalytical methods (Differential Scanning Calorimetry, DSC; Thermal Gravimetric Analysis, TGA). **Figure 36** shows the DSC (Mettler-Toledo 822e DSC) and **Figure 37** shows the TGA (Perkin Elmer TGA 6) curves measured in the range of 25°C to 300°C and 25°C to 300°C, respectively. The Crystal modification 1 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt shows a 0.18% weigh loss in the range of 25°C to 240°C. The DSC measurement gives a melting process with T_{onset,1}=212.6°C; and Tₚₑₐₖ=223.8°C.

The Crystal modification 1 of baricitinib p-toluenesulfonic acid salt can be prepared by a process comprising the steps of:
a/ dissolution of baricitinib free base in a suitable organic solvent;
b/ drop-wise addition of the p-toluenesulfonic acid aqueous solution;
c/ stirring the solution of the step b/ at 50°C for additional 1 hour;
d/ cooling the solution of the step c/ to room temperature;
e/ keeping the solution of the step d/ for 16 hours at room temperature while precipitation occurred;
f/ isolating the baricitinib p-toluenesulfonic acid salt in Crystal modification 1;
g/ optionally, drying of the product of step f/ at laboratory condition until the constant weight of the product is reached.

The suitable organic solvent is selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably tetrahydrofuran, even more preferably the suitable organic solvent is tetrahydrofuran at the temperature of 50°C.

Another process for the preparation of the Crystal modification 1 of baricitinib p-toluenesulfonic acid salt comprises the steps of:
a/ suspending baricitinib p-toluenesulfonic acid salt in tetrahydrofuran-water 95-5 V/V mixture at 50°C;
b/ stirring the suspension of step a/ at 50°C for 72 hours;
c/ isolating the Crystal modification 1 of baricitinib p-toluenesulfonic acid salt;
d/ optionally, drying of the product of step c/ at laboratory condition until the constant weight of the product is reached.

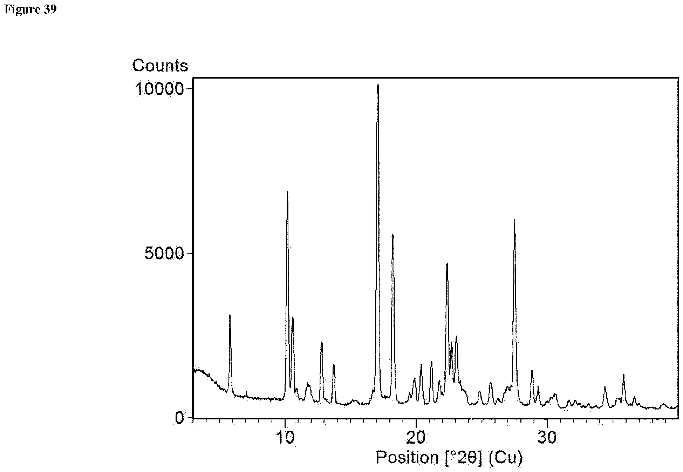

The Crystal modification 2 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt according to the invention has the characteristic XRPD pattern as shown in **Figure 39****.** XRPD pattern was recorded on an X-Ray Powder Diffractometer (X'PERT PRO MPD PANalytical). The Crystal modification 2 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt exhibits the following diffraction peaks in XRPD pattern, see **Table 8,** below:

**Table 8**

| **Pos. [°2θ]** | **d [Å]** | **Rel. Int. [%]** |
|---|---|---|
| 5.82 | 15.168 | 21.1 |
| 10.18 | 8.680 | 61.4 |
| 10.58 | 8.357 | 24.2 |
| 11.72 | 7.546 | 4.0 |
| 12.78 | 6.919 | 18.5 |
| 13.71 | 6.454 | 11.3 |
| 17.04 | 5.198 | 100.0 |
| 18.21 | 4.867 | 54.1 |
| 19.89 | 4.460 | 5.3 |
| 20.36 | 4.358 | 10.3 |
| 21.14 | 4.200 | 12.0 |
| 22.34 | 3.976 | 43.6 |
| 22.67 | 3.919 | 17.0 |
| 23.04 | 3.857 | 19.8 |
| 25.67 | 3.468 | 6.7 |
| 27.49 | 3.242 | 57.2 |
| 28.81 | 3.096 | 11.0 |
| 29.27 | 3.048 | 5.3 |
| 34.38 | 2.607 | 6.0 |
| 35.79 | 2.507 | 9.2 |

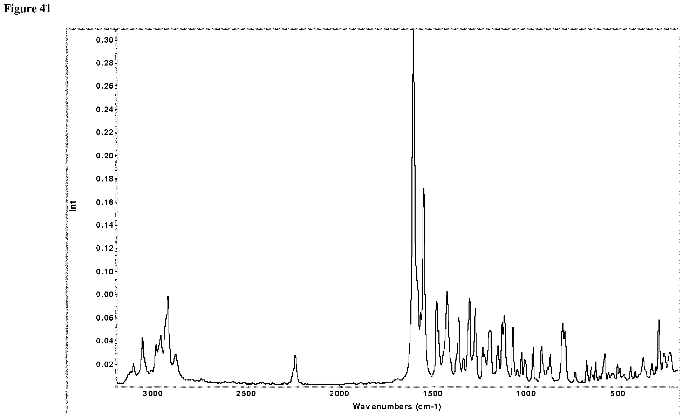

The Crystal modification 2 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt can be characterized by FTIR and Raman spectroscopy. **Figure 41** shows the FTIR spectrum (Nicolet Thermo 6700c) comprising characteristic peaks at 3113, 2929, 2817, 2360, 2254, 1624, 1120, 1008, 681 and 563 cm⁻¹ wavenumbers. The Crystal modification 2 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt is characterised by a Raman spectrum (Bruker RFS 100/S) comprising characteristic peaks at 3114, 3070, 2936, 2887, 2250, 1613, 1559, 1434, 1313 and 1081 cm⁻¹ wavenumbers, shown in **Figure 41****.**

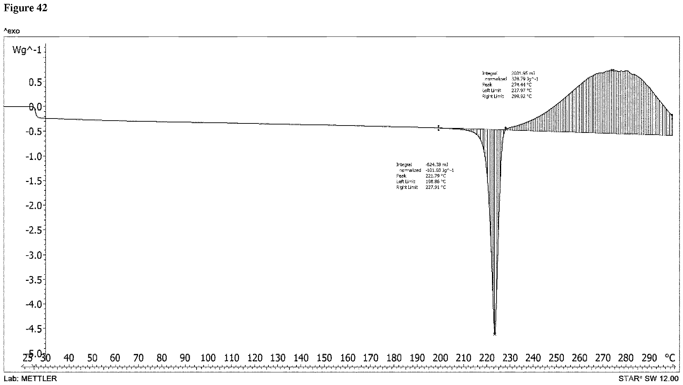

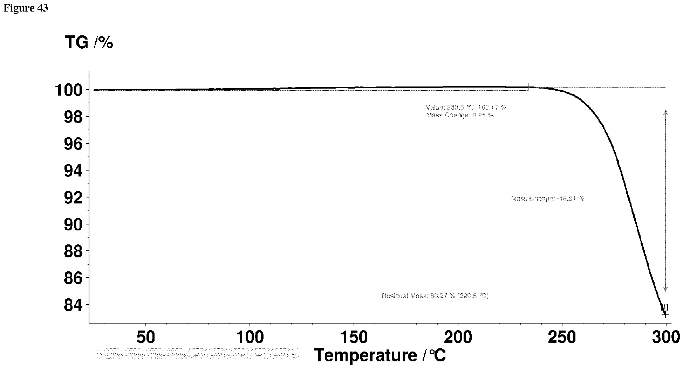

The Crystal modification 2 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt can be further described by thermal analytical methods. **Figure 42** shows the DSC (Mettler-Toledo 822e DSC) and **Figure 43** shows the TGA (NETZSCH TG 209 thermogravimetric analyser) curves measured in the range of 25°C to 300°C and 25°C to 300°C, respectively. The Crystal modification 2 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt shows a 0.25% weigh loss in the range of 25°C to 234°C. The DSC measurement gives a melting process with T_{onset,1}=198.9°C; and Tₚₑₐₖ=221.8°C.

The Crystal modification 2 of baricitinib p-toluenesulfonic acid salt can be prepared by a process comprising the steps of:
a/ suspending baricitinib p-toluenesulfonic acid salt in methanol at 50°C;
b/ stirring the suspension of step a/ at 50°C for 72 hours;
c/ isolating the Crystal modification 2 of baricitinib L-tartaric acid salt;
d/ optionally, drying of the product of step c/ at laboratory condition until the constant weight of the product is reached.

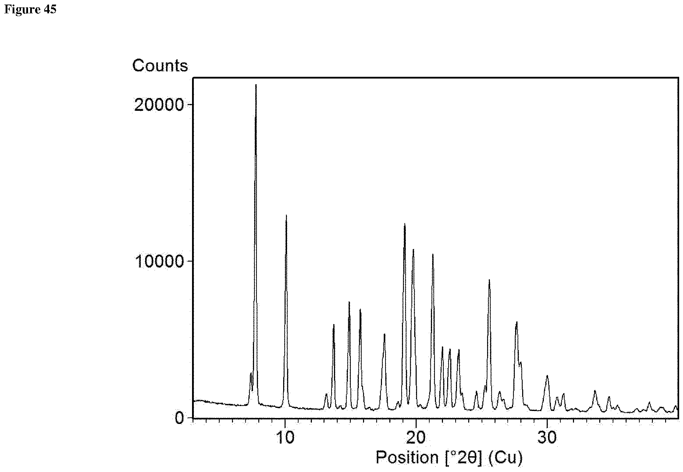

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile fumaric acid salt according to the invention has the characteristic XRPD pattern as shown in **Figure 45****.** XRPD pattern was recorded on an X-Ray Powder Diffractometer (X'PERT PRO MPD PANalytical). The Crystal modification 1 of 2-[1-Ethylsulfonyl-3-[4-(7H-pyrrolo[2,3-d]pyrimidin-4-yl)pyrazol-1-yl]azetidin-3-yl]acetonitrile fumaric acid salt exhibits the following diffraction peaks in XRPD pattern, see **Table 9,** below:

**Table 9**

| **Pos. [°2θ]** | **d [Å]** | **Rel. Int. [%]** |
|---|---|---|
| 7.74 | 11.408 | 100.0 |
| 10.07 | 8.777 | 61.3 |
| 13.69 | 6.465 | 26.0 |
| 14.86 | 5.956 | 33.3 |
| 15.72 | 5.634 | 31.0 |
| 17.57 | 5.043 | 21.7 |
| 19.08 | 4.648 | 60.7 |
| 19.78 | 4.485 | 47.2 |
| 21.23 | 4.181 | 51.2 |
| 21.99 | 4.039 | 20.2 |
| 22.57 | 3.936 | 17.8 |
| 23.19 | 3.832 | 19.7 |
| 24.57 | 3.621 | 5.9 |
| 25.54 | 3.485 | 44.4 |
| 26.38 | 3.376 | 4.7 |
| 27.61 | 3.228 | 30.4 |
| 30.00 | 2.976 | 10.7 |
| 30.72 | 2.908 | 4.7 |
| 31.18 | 2.866 | 6.2 |
| 33.62 | 2.663 | 7.1 |
| 34.66 | 2.586 | 5.5 |

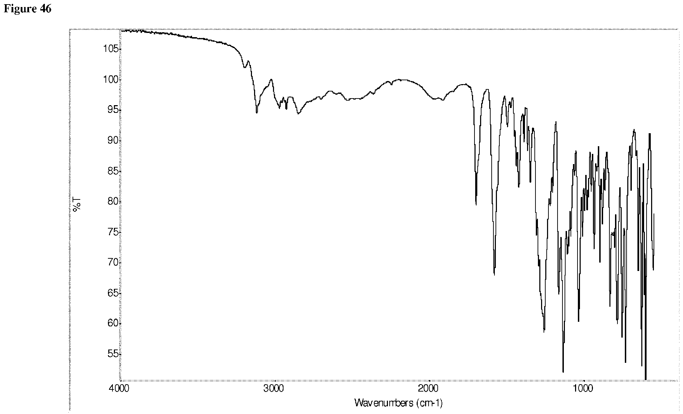

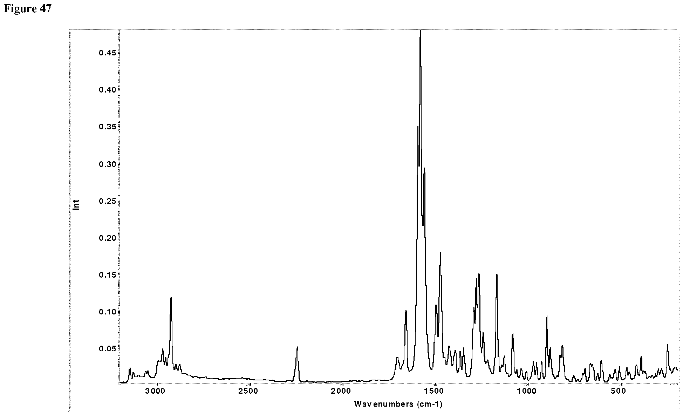

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile fumaric acid salt can be characterized by FTIR and Raman spectroscopy. **Figure 46** shows the FTIR spectrum (Nicolet Thermo 6700c) comprising characteristic peaks at 3197, 3119, 2972, 2927, 2248, 1701, 1582, 1261, 1138 and 734 cm⁻¹ wavenumbers. The Crystal modification 1 of 2-[1-Ethylsulfonyl-3-[4-(7H-pyrrolo[2,3-d]pyrimidin-4-yl)pyrazol-1-yl]azetidin-3-yl]acetonitrile fumaric acid salt is characterised by a Raman spectrum (Bruker RFS 100/S) comprising characteristic peaks at 3150, 3131, 2928, 2248, 1708, 1663, 1584, 1564, 1478 and 1174 cm⁻¹ wavenumbers, shown in **Figure 47****.**

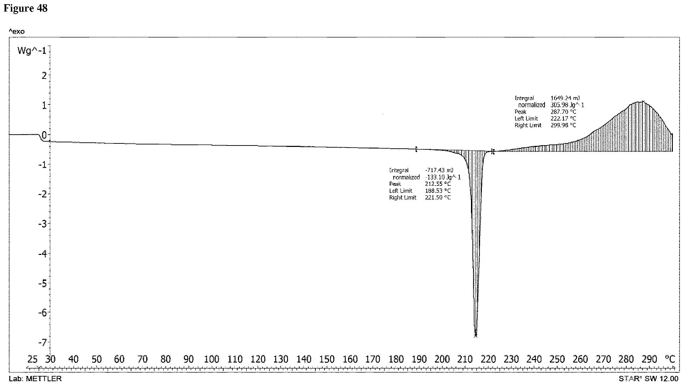

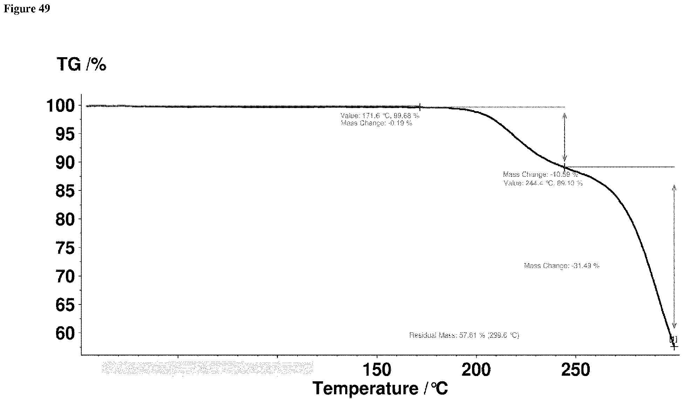

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile fumaric acid salt can be further described by thermal analytical methods. **Figure 48** shows the DSC (Mettler-Toledo 822e DSC) and **Figure 49** shows the TGA (NETZSCH TG 209 thermogravimetric analyser) curves measured in the range of 25°C to 300°C and 25°C to 300°C, respectively. The Crystal modification 1 of 2-[1-Ethylsulfonyl-3-[4-(7H-pyrrolo[2,3-d]pyrimidin-4-yl)pyrazol-1-yl]azetidin-3-yl]acetonitrile fumaric acid salt shows a 0.2% weigh loss in the range of 20°C to 172°C. The DSC measurement gives a melting process with T_{onset,1}=188.5°C; and Tₚₑₐₖ=212.6°C.

The Crystal modification 1 of baricitinib fumaric acid salt can be prepared by a process comprising the steps of:
a/ dissolution of baricitinib free base in a suitable organic solvent;
b/ drop-wise addition of the fumaric acid aqueous solution;
c/ stirring the solution of the step b/ at 50°C for additional 1 hour;
d/ cooling the solution of the step c/ to room temperature;
e/ keeping the solution of the step d/ for 16 hours at room temperature while precipitation occurred;
f/ isolating the baricitinib fumaric acid salt in Crystal modification 1;
g/ optionally, drying of the product of step f/ at laboratory condition until the constant weight of the product is reached.

The suitable organic solvent is selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably tetrahydrofuran, even more preferably the suitable organic solvent is tetrahydrofuran at the temperature of 50°C.

Another process for the preparation of the Crystal modification 1 of baricitinib fumaric acid salt comprises the steps of:
a/ suspending baricitinib fumaric acid salt in ethyl acetate at 50°C;
b/ stirring the suspension of step a/ at 50°C for 72 hours;
c/ isolating the Crystal modification 1 of baricitinib fumaric acid salt;
d/ optionally, drying of the product of step c/ at laboratory condition until the constant weight of the product is reached.

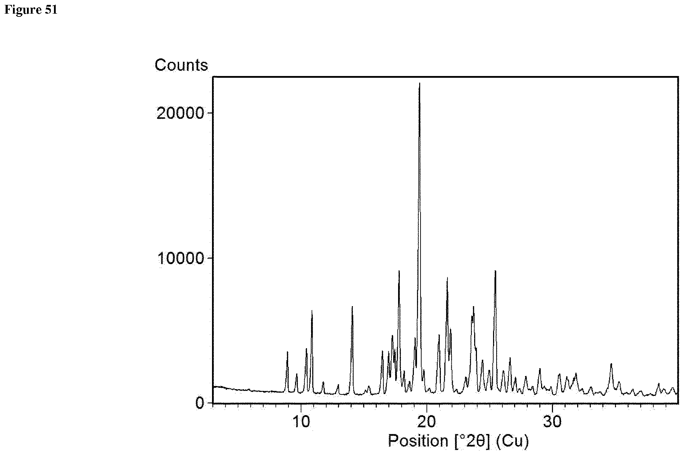

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile L-tartaric acid salt according to the invention has the characteristic XRPD pattern as shown in **Figure 51****.** XRPD pattern was recorded on an X-Ray Powder Diffractometer (X'PERT PRO MPD PANalytical). The Crystal modification 4 of 2-[1-Ethylsulfonyl-3-[4-(7H-pyrrolo[2,3-d]pyrimidin-4-yl)pyrazol-1-yl]azetidin-3-yl]acetonitrile L-tartaric acid salt exhibits the following diffraction peaks in XRPD pattern, see **Table 10,** below:

**Table 10**

| **Pos. [°2θ]** | **d [Å]** | **Rel. Int. [%]** |
|---|---|---|
| 8.89 | 9.943 | 13.5 |
| 9.63 | 9.180 | 6.9 |
| 10.39 | 8.508 | 15.1 |
| 10.84 | 8.155 | 25.9 |
| 14.05 | 6.300 | 29.2 |
| 16.43 | 5.392 | 13.9 |
| 16.94 | 5.229 | 13.3 |
| 17.24 | 5.139 | 19.3 |
| 17.77 | 4.988 | 41.3 |
| 19.38 | 4.576 | 100.0 |
| 20.91 | 4.244 | 18.3 |
| 21.59 | 4.113 | 35.1 |
| 21.87 | 4.061 | 21.8 |
| 23.56 | 3.774 | 27.9 |
| 24.39 | 3.647 | 11.0 |
| 24.92 | 3.571 | 7.9 |
| 25.43 | 3.499 | 44.3 |
| 26.06 | 3.417 | 8.2 |
| 26.58 | 3.351 | 12.1 |
| 27.00 | 3.300 | 5.1 |
| 27.84 | 3.202 | 6.2 |
| 28.93 | 3.083 | 7.9 |
| 30.48 | 2.930 | 6.6 |
| 31.12 | 2.872 | 6.0 |
| 31.83 | 2.809 | 6.8 |
| 34.63 | 2.588 | 11.8 |

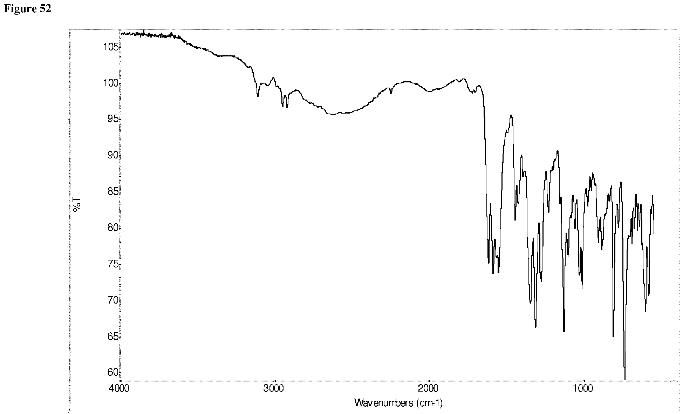

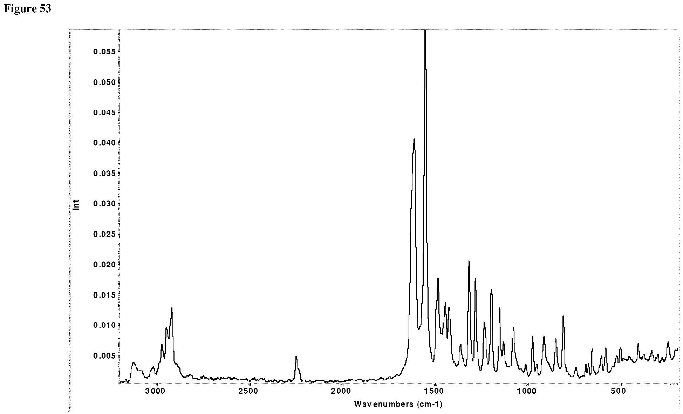

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile L-tartaric acid salt can be characterized by FTIR and Raman spectroscopy. **Figure 52** shows the FTIR spectrum (Nicolet Thermo 6700c) comprising characteristic peaks at 3111, 2951, 2921, 2252, 1620, 1592, 1556, 1317, 1133, 740 cm⁻¹ wavenumbers. The Crystal modification 1 of 2-[1-Ethylsulfonyl-3-[4-(7H-pyrrolo[2,3-d]pyrimidin-4-yl)pyrazol-1-yl]azetidin-3-yl]acetonitrile L-tartaric acid salt is characterised by a Raman spectrum (Bruker RFS 100/S) comprising characteristic peaks at 3130, 2976, 2953, 2923, 2253, 1617, 1559, 1489, 1323 and 815 cm⁻¹ wavenumbers, shown in **Figure 53****.**

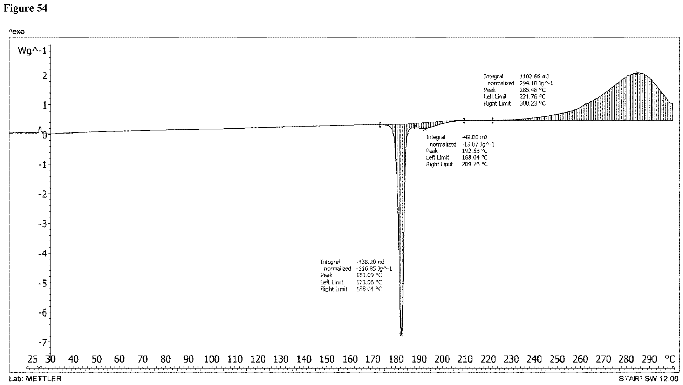

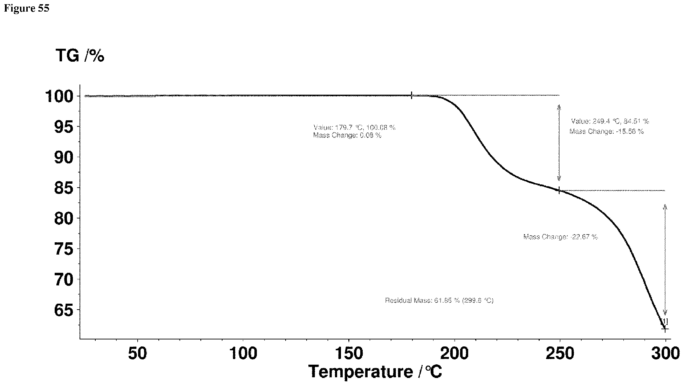

The Crystal modification 1 of 2-[1-Ethylsulfonyl-3-[4-(7H-pyrrolo[2,3-d]pyrimidin-4-yl)pyrazol-1-yl]azetidin-3-yl]acetonitrile L-tartaric acid salt can be further described by thermal analytical methods. **Figure 54** shows the DSC (Mettler-Toledo 822e DSC) and **Figure 55** shows the TGA (NETZSCH TG 209 thermogravimetric analyser) curves measured in the range of 25°C to 300°C and 25°C to 300°C, respectively. The Crystal modification 1 of 2-[1-Ethylsulfonyl-3-[4-(7H-pyrrolo[2,3-d]pyrimidin-4-yl)pyrazol-1-yl]azetidin-3-yl]acetonitrile L-tartaric acid salt shows a 0.1% weigh loss in the range of 20°C to 172°C. The DSC measurement gives a melting process with T_{onset,1}=173.1°C; and Tₚₑₐₖ=181.1°C.

In one of the aspects of the invention, an alternative process for preparation of the Crystal modification 1 of 2-[1-Ethylsulfonyl-3-[4-(7H-pyrrolo[2,3-d]pyrimidin-4-yl)pyrazol-1-yl]azetidin-3-yl]acetonitrile L-tartaric acid salt is provided. The process for the preparation of the Crystal modification 1 comprises the steps of
a/ dissolution of baricitinib free base in a suitable organic solvent;
b/ drop-wise addition of the L-tartaric acid aqueous solution;
c/ stirring the solution of the step b/ at 50°C for additional 1 hour;
d/ cooling the solution of the step c/ to room temperature;
e/ keeping the solution of the step d/ for 16 hours at room temperature while precipitation occurred;
f/ isolating the baricitinib L-tartaric acid salt in Crystal modification 1;
g/ optionally, drying of the product of step f/ at laboratory condition until the constant weight of the product is reached.

The suitable organic solvent is selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably tetrahydrofuran, even more preferably the suitable organic solvent is tetrahydrofuran at the temperature of 50°C.

Another process for the preparation of the Crystal modification 1 of baricitinib L-tartaric acid salt comprises the steps of:
a/ suspending baricitinib L-tartaric salt in acetone at 50°C;
b/ stirring the suspension of step a/ at 50°C for 72 hours;
c/ isolating the Crystal modification 1 of baricitinib L-tartaric acid salt;
d/ optionally, drying of the product of step c/ at laboratory condition until the constant weight of the product is reached.

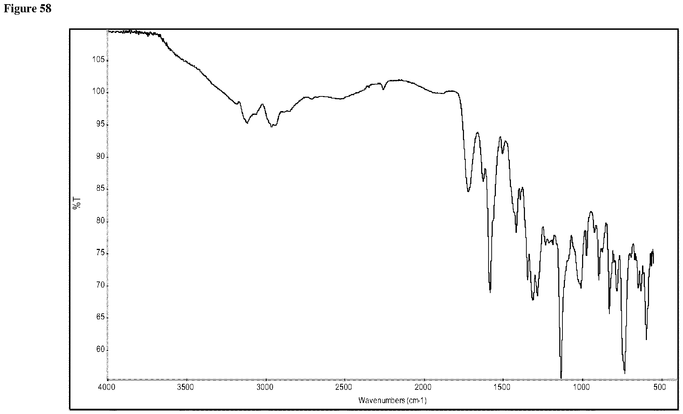

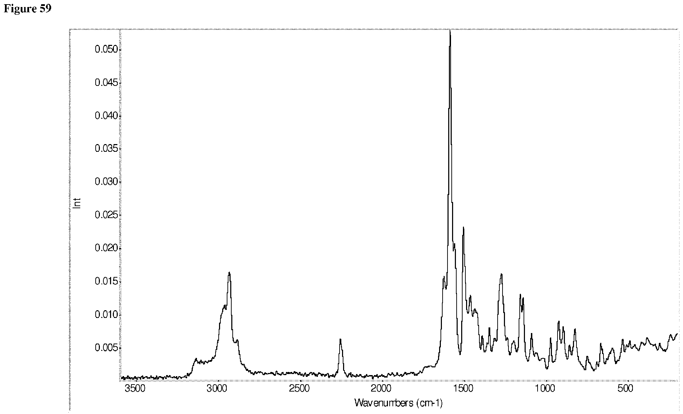

The amorphous citric acid salt of 2-[1-Ethylsulfonyl-3-[4-(7H-pyrrolo[2,3-d]pyrimidin-4-yl)pyrazol-1-yl]azetidin-3-yl]acetonitrile can be characterized by FTIR and Raman spectroscopy. **Figure 58** shows the FTIR spectrum (Nicolet Thermo 6700c) comprising characteristic peaks at 3118, 2964, 2257, 1720, 1626, 1581, 1417, 1135, 733 and 596 cm⁻¹ wavenumbers. The amorphous citric acid salt of 2-[1-Ethylsulfonyl-3-[4-(7H-pyrrolo[2,3-d]pyrimidin-4-yl)pyrazol-1-yl]azetidin-3-yl]acetonitrile is characterised by a Raman spectrum (Bruker RFS 100/S) comprising characteristic peaks at 3139, 2964, 2935, 2887, 2259, 1625, 1589, 1507, 1275 and 825cm⁻¹ wavenumbers, shown in **Figure 59****.**

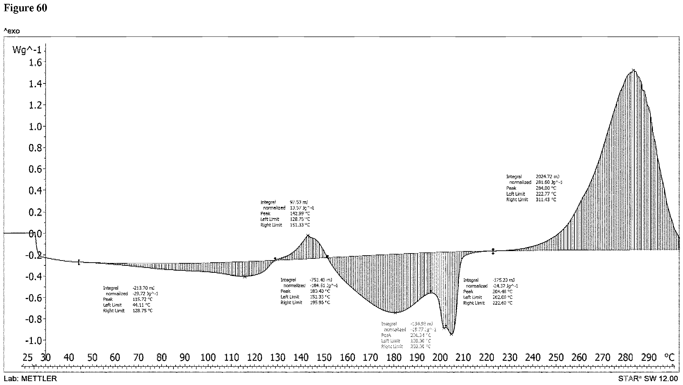

The amorphous citric acid salt of 2-[1-Ethylsulfonyl-3-[4-(7H-pyrrolo[2,3-d]pyrimidin-4-yl)pyrazol-1-yl]azetidin-3-yl]acetonitrile can be further described by thermal analytical methods. **Figure 60** shows the DSC (Mettler-Toledo 822e DSC) curve measured in the range of 25°C to 300°C. The DSC measurement gives a melting process with T_{onset,1}=44.1 C (corresponding to the solvent loss) and T_{onset,recrystallization}=128.8 °C.

In one of the aspects of the invention, an alternative process for preparation of the amorphous citric acid salt of 2-[1-Ethylsulfonyl-3-[4-(7H-pyrrolo[2,3-d]pyrimidin-4-yl)pyrazol-1-yl]azetidin-3-yl]acetonitrile is provided. The process for the preparation comprises the steps of
a/ dissolution of baricitinib free base in a suitable organic solvent at 50°C;
b/ drop-wise addition of the citric acid aqueous solution;
c/ stirring the solution of the step b/ at 50°C for additional 1 hour;
d/ cooling the solution of the step c/ to room temperature;
e/ keeping the solution of the step d/ for 2 hours at room temperature;
f/ evaporating the solvent of the solution from step d/ till complete dryness;
g/ isolating the amorphous baricitinib citric acid salt;
h/ optionally, drying of the product of step f/ at laboratory condition until the constant weight of the product is reached.

The term "room temperature" is defined as a temperature between 15°C and 29°C for the purpose of this document; preferably it is between 20-23°C.

The term "drying at laboratory condition", as used in this patent application, means drying at room temperature and relative humidity 20-60%.

### Analysis - XRPD (X-Ray Powder Diffractometry)

Diffractograms were obtained with laboratory X'PERT PRO MPD PANalytical diffractometer, used radiation CuKα (λ = 1.542 Å).

### Generator settings:

- excitation voltage 45 kV
- anodic current 40 mA.

### Scan description:

- scan type - gonio
- measurement range 2 - 40° 2θ
- step size 0.01° 2θ
- step time: 0.5 s.

Samples were measured as received on Si plate (zero background holder).

Incident beam optics: programmable divergence slits (irradiated length 10 mm). 10 mm mask. 1/4° anti-scatter fixed slit, 0.02 rad Soller slits.

Diffracted beam optics: X'Celerator detector, scanning mode, active length 2.122°. 0.02 rad Soller slits, anti-scatter slit 5.0 mm. Ni filter.

### Analysis - FTIR (Fourier-Transformed Infra-Red) spectroscopy

FTIR spectrum were recorded by Nicolet Thermo 6700 spectrometer.

### General settings:

Number of sample scans: 45
Number of background scans: 45
Resolution: 4.000
Sample gain: 4.0
Optical velocity: 0.6329
Aperture: 100.00

### Analysis - Raman spectroscopy

Raman spectrum were recorded by FT-Raman Bruker RFS 100/S Spectrometer

### General settings:

Excitation source: Nd-YAG laser (1064 nm)
Applied spectrum domain: 4000-200 cm⁻¹
Applied laser power: 250 mW
Detector: liquid nitrogen cooled Ge-diode detector (D418-T)
Resolution: 4 cm⁻¹
Number of accumulations: 128
Scattering geometry: 180° (back scattering)
Aperture: 3.5 mm

### Analysis - DSC (Differential Scanning Calorimetry)

DSC measurements were performed using a Mettler-Toledo 822e DSC.

Samples were placed into standard aluminum pans (40 µL) sealed with a pierced lid. The sample cell was heated under a nitrogen purge at a rate of 10 °C/min from 25 °C up to a final temperature of 300 °C with 50 mL/min nitrogen purge. The temperatures specified in relation to DSC analyses are the temperatures of the peak maxima (Tₚₑₐₖ) and onset temperature (Tₒₙₛₑₜ) of peaks for the crystalline form. The enthalpy is given in J/g.

The weight sample was about 2.5-3 mg.

### Analysis - TGA (ThermoGravimetric Analysis)

TGA analyses were performed using a NETZSCH TG 209 thermogravimetric analyser (NETZSCH-Geratebau GmbH, Germany).

Each sample was placed in an aluminum sample pan and inserted into the TG furnace. The furnace was heated under nitrogen purge at a rate of 10 °C/min from 25 °C up to a final temperature of 300 °C.

The weight sample was about 5-15 mg.

### Examples

The following examples are intended to further illustrate the present invention without limiting its scope.

### Example 1

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrobromic acid salt

1g of baricitinib free base (2.7 mmol) was dissolved in 20 mL tetrahydrofuran by heating to 50°C applying a continuous stirring and 0.366 mL of hydrobromic acid (3.2 mmol; 48% aqueous solution) was drop-wise added. The suspension was slowly cooled down to room temperature while precipitation occurred. The solution was stirred at ambient conditions overnight. The solids were collected by filtration and dried by vacuum suction at laboratory condition.

**Product:** 1.22 g (2.62 mmol) white crystalline solid, HPLC: 99.7%
The bromide content determined by titration is 17.1 % Br-=1 molar equivalent.
XRPD pattern was measured (**Figure 1**) and showed that the compound is in a crystalline state that was designated as Crystal modification 1 of baricitinib hydrobromic acid salt.

### Example 2

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrobromic acid salt

1g of baricitinib free base (2.7 mmol) was dissolved in 25 mL 2-propanol by heating to 80°C applying a continuous stirring and 0.366 mL of hydrobromic acid (3.2 mmol; 48% aqueous solution) was drop-wise added. The suspension was slowly cooled down to room temperature while precipitation occurred. The solution was stirred at ambient conditions overnight. The solids were collected by filtration and dried by vacuum suction at laboratory condition.

**Product:** 1.2 g (2.6 mmol) white crystalline solid, HPLC: 99.7%
The bromide content determined by titration is 17.1 % Br-=1 molar equivalent.
XRPD pattern was measured (**Figure 1**) and showed that the compound is in a crystalline state that was designated as Crystal modification 1 of baricitinib hydrobromic acid salt.

### Example 3

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrobromic acid salt

1g of baricitinib hydrobromide (2.21 mmol) was suspended in 10 mL 2-propanol at 50°C applying continuous stirring. The suspension was stirred at 50°C for 72 hours. The solids were collected by filtration and dried by vacuum suction at laboratory condition.

**Product:** 0.99 g (2.2 mmol) white crystalline solid, HPLC: 99.7%
The bromide content determined by titration is 17.1 % Br-=1 molar equivalent.
XRPD pattern was measured and showed that the compound is in a crystalline state that was designated as Crystal modification 1 of baricitinib hydrobromic acid salt.

### Example 4

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt

1g of baricitinib free base (2.7 mmol) was dissolved in 20 mL tetrahydrofuran by heating to 50°C applying a continuous stirring and 0.281 mL of hydrochloric acid (3.2 mmol; 35% aqueous solution) was drop-wise added. The solution was slowly cooled down to room temperature while precipitation occurred. The suspension was stirred at ambient conditions overnight. The solids were collected by filtration and dried by vacuum suction at laboratory condition.

**Product:** 1.01 g (2.45 mmol) white crystalline solid, HPLC: 99.7%
The chloride content determined by titration is 8.6% Br-=1 molar equivalent.
XRPD pattern (**Figure 6**) was measured and showed that the compound is in a crystalline state that was designated as Crystal modification 1 of baricitinib hydrochloric acid salt.

### Example 5

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt

1g of baricitinib free base (2.7 mmol) was dissolved in 15 mL methanol-water 1-1 V/V mixture by heating to 65°C applying a continuous stirring and 0.281 mL of hydrochloric acid (3.2 mmol; 35% aqueous solution) was drop-wise added. The solution was slowly cooled down to room temperature while precipitation occurred. The suspension was stirred at ambient conditions overnight. The solids were collected by filtration and dried by vacuum suction at laboratory condition.

**Product:** 0.95 g (2.31 mmol) white crystalline solid, HPLC: 99.7%
The chloride content determined by titration is 8.6% Br-=1 molar equivalent.
XRPD pattern (**Figure 6**) was measured and showed that the compound is in a crystalline state that was designated as Crystal modification 1 of baricitinib hydrochloric acid salt.

### Example 6

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile hydrochloric acid salt

1g of baricitinib hydrochloride (2.45 mmol) was suspended in 10 mL acetone at 50°C applying continuous stirring. The suspension was stirred at 50°C for 72 hours. The solids were collected by filtration and dried by vacuum suction at laboratory condition.

**Product:** 0.95 g (2.33 mmol) white crystalline solid, HPLC: 99.7%
The chloride content determined by titration is 8.6% Br-=1 molar equivalent.
XRPD pattern was measured (**Figure 11**) and showed that the compound is in a crystalline state that was designated as Crystal modification 1 of baricitinib hydrochloric acid salt.

### Example 7

### Preparation of Crystal modification 2 of 2-[1-Ethylsulfonyl-3-[4-(7H-pyrrolo[2,3-d]pyrimidin-4-yl)pyrazol-1-yl]azetidin-3-yllacetonitrile hydrochloric acid salt

1g of baricitinib hydrochloride (2.45 mmol) was suspended in 10 mL water at 50°C applying continuous stirring. The solution was stirred at 50°C for 72 hours. The solids were collected by filtration and dried by vacuum suction at laboratory condition.

**Product:** 0.90 g (2.21 mmol) white crystalline solid, HPLC: 99.8%
The chloride content determined by titration is 8.6% Br-=1 molar equivalent.
XRPD pattern (**Figure 11**) was measured and showed that the compound is in a crystalline state that was designated as Crystal modification 2 of baricitinib hydrochloric acid salt.

### Example 8

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile sulfuric acid salt

1g of baricitinib free base (2.7 mmol) was dissolved in 20 mL tetrahydrofuran by heating to 50°C applying a continuous stirring and 0.179 mL of sulfuric acid (3.2 mmol; 96% aqueous solution) was drop-wise added. The solution was slowly cooled down to room temperature while precipitation occurred. The suspension was stirred at ambient conditions overnight. The solids were collected by filtration and dried by vacuum suction at laboratory condition.

**Product:** 1.26 g (2.7 mmol) white crystalline solid, HPLC: 98.2%
The sulfate content determined by titration is 22.2% Br-=1.1 molar equivalent.
XRPD pattern (**Figure 16**) was measured and showed that the compound is in a crystalline state that was designated as Crystal modification 1 of baricitinib sulfuric acid salt.

### Example 9

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile sulfuric acid salt

1g of baricitinib free base (2.7 mmol) was dissolved in 28 mL acetonitrile by heating to 80°C applying a continuous stirring and 0.179 mL of sulfuric acid (3.2 mmol; 96% aqueous solution) was drop-wise added. The solution was slowly cooled down to room temperature while precipitation occurred. The suspension was stirred at ambient conditions overnight. The solids were collected by filtration and dried by vacuum suction at laboratory condition.

**Product:** 1.08 g (2.32 mmol) white crystalline solid, HPLC: 98.1%
The sulfate content determined by titration is 22.2% Br-=1.1 molar equivalent.
XRPD pattern (**Figure 16**) was measured and showed that the compound is in a crystalline state that was designated as Crystal modification 1 of baricitinib sulfuric acid salt.

### Example 10

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile sulfuric acid salt

1g of baricitinib hydrobromide (2.13 mmol) was suspended in 10 mL ethyl acetate at 50°C applying continuous stirring. The suspension was stirred at 50°C for 72 hours. The solids were collected by filtration and dried by vacuum suction at laboratory condition.

**Product:** 0.99 g (2.11 mmol) white crystalline solid, HPLC: 98.5%
The sulfate content determined by titration is 22.2% Br-=1.1 molar equivalent.
XRPD pattern was measured and showed that the compound is in a crystalline state that was designated as Crystal modification 1 of baricitinib sulfuric acid salt.

### Example 11

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile methanesulfonic acid salt

1g of baricitinib free base (2.7 mmol) was dissolved in 20 mL tetrahydrofuran by heating to 50°C applying a continuous stirring and 0.214 mL of methanesulfonic acid (3.2 mmol; >98% aqueous solution) was drop-wise added. The solution was slowly cooled down to room temperature while precipitation occurred. The suspension was stirred at ambient conditions overnight. The solids were collected by filtration and dried by vacuum suction at laboratory condition.

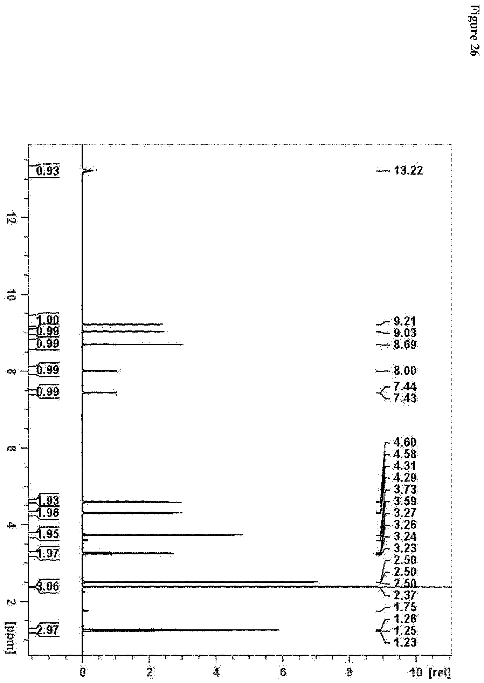

**Product:** 0.95 g (2.0 mmol) white crystalline solid, HPLC: 99.7%
The stoichiometry determined by ¹H NMR (**Figure 26**) is 1:1. XRPD pattern (**Figure 21**) was measured and showed that the compound is in a crystalline state that was designated as Crystal modification 1 of baricitinib methanesulfonic acid salt. Crystal X-ray diffraction resulted in the unit cell parameters summarized in Table 11.

**Table 11**

| **Crystal unit cell parameters** | **Baricitinib mesylate Modification 1** |
|---|---|
| Formula | C₁₇H₂₁N₇O₅S₂ |
| Cell parameters | a=16.7330(2) Å |
| | b=7.10319(6) Å |
| | c=19.0201(3) Å |
| | α=90° |
| | β=115.817(17)° |
| | γ=90° |
| Volume | 2035.05 Å³ |
| Crystal system | Monoclinic |
| Space group | *P* -2₁/c |
| R-factor | 0.0291 |
| Density | 1.526 g/cm³ |
| Temperature | 120 K |
| Z | 4 |

### Example 12

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile methanesulfonic acid salt

1g of baricitinib free base (2.7 mmol) was dissolved in 15 mL methanol-water 1-1 V/V mixture by heating to 65°C applying a continuous stirring and 0.214 mL of methanesulfonic acid (3.2 mmol; >98% aqueous solution) was drop-wise added. The solution was slowly cooled down to room temperature while precipitation occurred. The suspension was stirred at ambient conditions overnight. The solids were collected by filtration and dried by vacuum suction at laboratory condition.

**Product:** 1.05 g (2.21 mmol) white crystalline solid, HPLC: 99.8%
The stoichiometry determined by ¹H NMR (**Figure 26**) is 1:1. XRPD pattern (**Figure 21**) was measured and showed that the compound is in a crystalline state that was designated as Crystal modification 1 of baricitinib methanesulfonic acid salt. Crystal X-ray diffraction resulted in the unit cell parameters summarized in Table 11.

### Example 13

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile methanesulfonic acid salt

1g of baricitinib mesylate (2.13 mmol) was suspended in 10 mL ethanol at 50°C applying continuous stirring. The suspension was stirred at 50°C for 72 hours. The solids were collected by filtration and dried by vacuum suction at laboratory condition.

**Product:** 0.95 g (2.0 mmol) white crystalline solid, HPLC: 99.7%
The stoichiometry determined by ¹H NMR is 1:1.
XRPD pattern was measured and showed that the compound is in a crystalline state that was designated as Crystal modification 1 of baricitinib methanesulfonic acid salt.

### Example 14

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile ethanesulfonic acid salt

1g of baricitinib free base (2.7 mmol) was dissolved in 20 mL tetrahydrofuran by heating to 50°C applying a continuous stirring and 0.377 mL of ethanesulfonic acid (3.2 mmol; 70% aqueous solution) was drop-wise added. The solution was slowly cooled down to room temperature while precipitation occurred. The suspension was stirred at ambient conditions overnight. The solids were collected by filtration and dried by vacuum suction at laboratory condition.

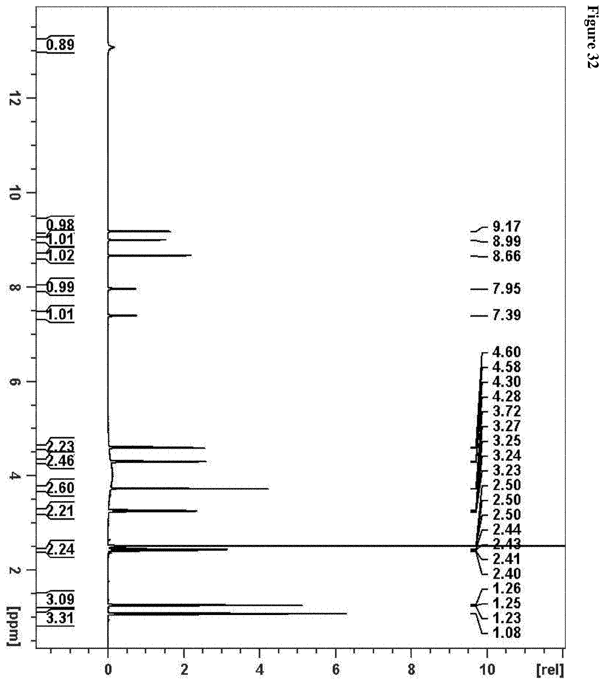

**Product:** 1.228 g (2.55 mmol) white crystalline solid, HPLC: 99.9%
The stoichiometry determined by ¹H NMR (**Figure 32**) is 1:1.
XRPD pattern (**Figure 27**) was measured and showed that the compound is in a crystalline state that was designated as Crystal modification 1 of baricitinib ethanesulfonic acid salt.

### Example 15

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile ethanesulfonic acid salt

1g of baricitinib free base (2.7 mmol) was dissolved in 28 mL acetonitrile by heating to 80°C applying a continuous stirring and 0.377 mL of ethanesulfonic acid (3.2 mmol; 70% aqueous solution) was drop-wise added. The solution was slowly cooled down to room temperature while precipitation occurred. The suspension was stirred at ambient conditions overnight. The solids were collected by filtration and dried by vacuum suction at laboratory condition.

**Product:** 1.22 g (2.54 mmol) white crystalline solid, HPLC: 99.9%
The stoichiometry determined by ¹H NMR (**Figure 32**) is 1:1.
XRPD pattern (**Figure 27**) was measured and showed that the compound is in a crystalline state that was designated as Crystal modification 1 of baricitinib ethanesulfonic acid salt.

### Example 16

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile ethanesulfonic acid salt

1g of baricitinib esylate (2.1 mmol) was suspended in 10 mL ethyl acetate at 50°C applying continuous stirring. The suspension was stirred at 50°C for 72 hours. The solids were collected by filtration and dried by vacuum suction at laboratory condition.

**Product:** 0.97 g (2.0 mmol) white crystalline solid, HPLC: 99.9%
The stoichiometry determined by ¹H NMR is 1:1.
XRPD pattern was measured and showed that the compound is in a crystalline state that was designated as Crystal modification 1 of baricitinib ethanesulfonic acid salt.

### Example 17

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt

1g of baricitinib free base (2.7 mmol) was dissolved in 15 mL tetrahydrofuran by heating to 50°C applying a continuous stirring and 672 mg of p-toluenesulfonic acid (3.2 mmol) dissolved in 1 mL water was drop-wise added. The solution was slowly cooled down to room temperature while precipitation occurred. The suspension was stirred at ambient conditions overnight. The solids were collected by filtration and dried by vacuum suction at laboratory condition.

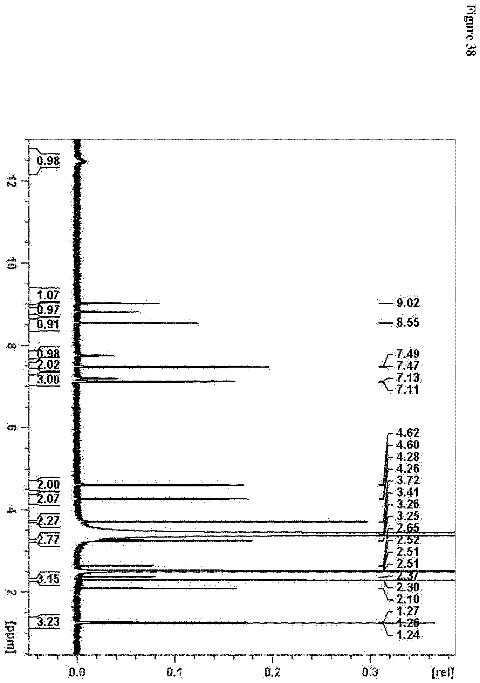

**Product:** 1.03 g (1.83 mmol) white crystalline solid, HPLC: 99.9%
The stoichiometry determined by ¹H NMR (**Figure 38** is 1:1. XRPD pattern (**Figure 33**) was measured and showed that the compound is in a crystalline state that was designated as Crystal modification 1 of baricitinib p-toluenesulfonic acid salt.
Crystal X-ray diffraction resulted in the unit cell parameters summarized in Table 12.

**Table 12**

| **Crystal unit cell parameters** | **Baricitinib tosylate Modification 1** |
|---|---|
| Formula | C₂₃H₂₅N₇O₅S₂ |
| Cell parameters | a=9.0391(13) Å |
| | b=30.734(3) Å |
| | c=9.7357(15) Å |
| | α=90° |
| | β=115.244(18) ° |
| | γ=90° |
| Volume | 2446.37 Å³ |
| Crystal system | Monoclinic |
| Space group | *P* -2₁/c |
| R-factor | 0.0279 |
| Density | 1.476 g/cm³ |
| Temperature | 120 K |
| Z | 4 |

### Example 18

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt

1g of baricitinib free base (2.7 mmol) was dissolved in 25 mL ethyl acetate by heating to 75°C applying a continuous stirring and 672 mg of p-toluenesulfonic acid (3.2 mmol) dissolved in 1 mL water was drop-wise added. The solution was slowly cooled down to room temperature while precipitation occurred. The suspension was stirred at ambient conditions overnight. The solids were collected by filtration and dried by vacuum suction at laboratory condition.

**Product:** 1.13 g (2.01 mmol) white crystalline solid, HPLC: 99.9%
The stoichiometry determined by ¹H NMR (**Figure 38** is 1:1. XRPD pattern (**Figure 33**) was measured and showed that the compound is in a crystalline state that was designated as Crystal modification 1 of baricitinib p-toluenesulfonic acid salt.

### Example 19

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt

1g of baricitinib tosylate (1.78 mmol) was suspended in 10 mL tetrahydrofuran-water 95-5 V/V mixture at 50°C applying continuous stirring. The suspension was stirred at 50°C for 72 hours. The solids were collected by filtration and dried by vacuum suction at laboratory condition.

**Product:** 0.99 g (1.77 mmol) white crystalline solid, HPLC: 99.9%
The stoichiometry determined by ¹H NMR is 1:1.
XRPD pattern was measured and showed that the compound is in a crystalline state that was designated as Crystal modification 1 of baricitinib p-toluenesulfonic acid salt.

### Example 20

### Preparation of Crystal modification 2 of 2-[1-Ethylsulfonyl-3-[4-(7H-pyrrolo[2,3-d]pyrimidin-4-yl)pyrazol-1-yl]azetidin-3-yl]acetonitrile p-toluenesulfonic acid salt

1g of baricitinib tosylate (1.78 mmol) was suspended in 10 mL methanol at 50°C applying continuous stirring. The suspension was stirred at 50°C for 72 hours. The solids were collected by filtration and dried by vacuum suction at laboratory condition.

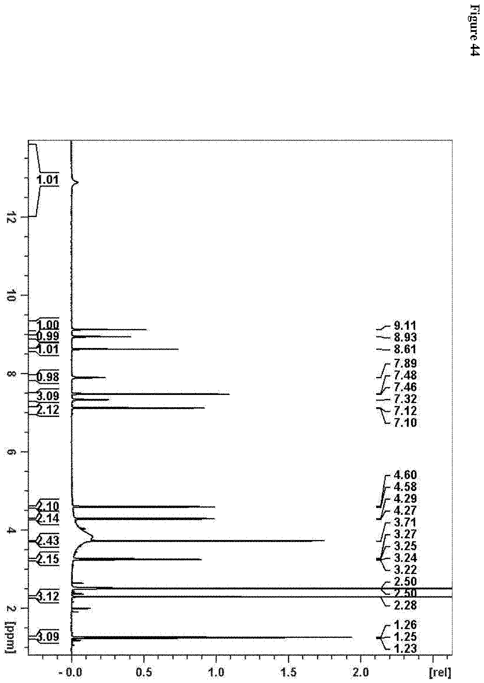

Product: 0.92 g (1.64 mmol) white crystalline solid, HPLC: 99.9%
The stoichiometry determined by ¹H NMR (**Figure 44**) is 1:1.
XRPD pattern was measured (**Figure 39**) and showed that the compound is in a crystalline state that was designated as Crystal modification 2 of baricitinib p-toluenesulfonic acid salt.

### Example 21

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile fumaric acid salt

1g of baricitinib free base (2.7 mmol) was dissolved in 15 mL tetrahydrofuran by heating to 50°C applying a continuous stirring and 188 mg of fumaric acid (1.62 mmol) dissolved in 2.5 mL water at 100°C was drop-wise added. The solution was slowly cooled down to room temperature while precipitation occurred. The suspension was stirred at ambient conditions overnight. The solids were collected by filtration and dried by vacuum suction at laboratory condition.

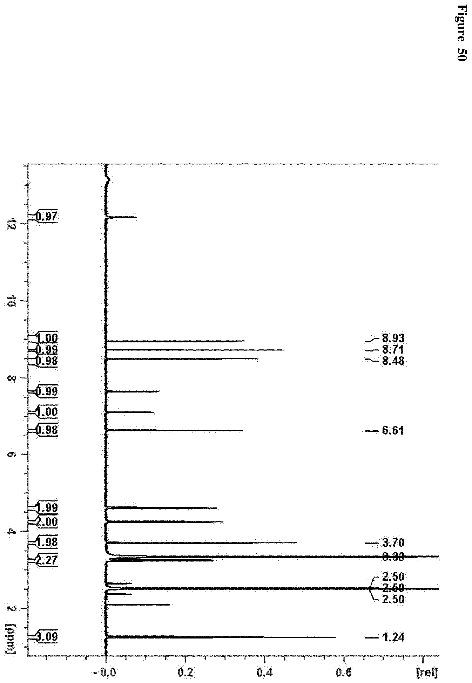

**Product:** 1.02 g (2.38 mmol) white crystalline solid, HPLC: 99.99%
The stoichiometry determined by ¹H NMR (**Figure 50**) is API: fumaric acid 2:1.
XRPD pattern (**Figure 45**) was measured and showed that the compound is in a crystalline state that was designated as Crystal modification 1 of baricitinib fumaric acid salt.

### Example22

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile fumaric acid salt

1g of baricitinib fumarate (2.33 mmol) was suspended in 10 mL ethyl acetate at 50°C applying continuous stirring. The suspension was stirred at 50°C for 72 hours. The solids were collected by filtration and dried by vacuum suction at laboratory condition.

**Product:** 0.94 g (2.19 mmol) white crystalline solid, HPLC: 99.99%
The stoichiometry determined by ¹H NMR is API: fumaric acid 2:1.
XRPD pattern was measured and showed that the compound is in a crystalline state that was designated as Crystal modification 1 of baricitinib fumaric acid salt.

### Example 23

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile L-tartaric acid salt

1g of baricitinib free base (2.7 mmol) was dissolved in 15 mL tetrahydrofuran by heating to 50°C applying a continuous stirring and 243 mg of L-tartaric acid (1.62 mmol) dissolved in 0.5 mL water at was drop-wise added. The solution was slowly cooled down to room temperature while precipitation occurred. The suspension was stirred at ambient conditions overnight. The solids were collected by filtration and dried by vacuum suction at laboratory condition.

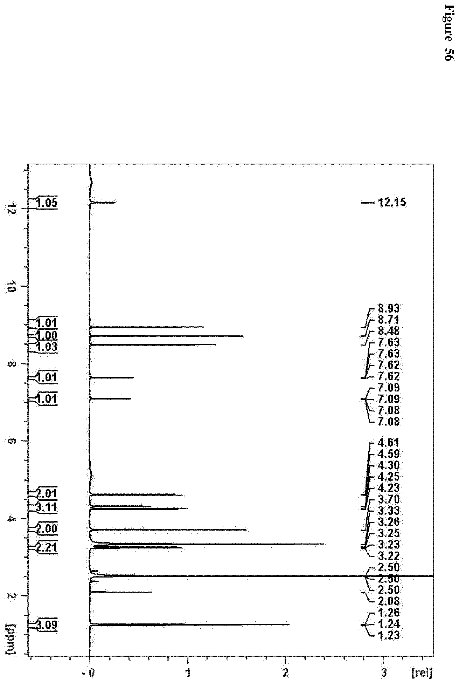

**Product:** 1.13 g (2.53 mmol) white crystalline solid, HPLC: 99.99%
The stoichiometry determined by ¹H NMR (**Figure 56**) is API: L-tartaric acid 2:1.
XRPD pattern (**Figure 51**) was measured and showed that the compound is in a crystalline state that was designated as Crystal modification 1 of baricitinib L-tartaric acid salt.

### Example 24

### Preparation of Crystal modification 1 of 2-[1-Ethylsulfonyl-3-[4-(7H-pyrrolo[2,3-d]pyrimidin-4-yl)pyrazol-1-yl]azetidin-3-yl]acetonitrile L-tartaric acid salt

1g of baricitinib L-tartrate (2.24 mmol) was suspended in 10 mL acetone at 50°C applying continuous stirring. The suspension was stirred at 50°C for 72 hours. The solids were collected by filtration and dried by vacuum suction at laboratory condition.

**Product:** 0.97 g (2.17 mmol) white crystalline solid, HPLC: 99.99%
The stoichiometry determined by ¹H NMR is API: L-tartaric acid 2:1.
XRPD pattern was measured and showed that the compound is in a crystalline state that was designated as Crystal modification 1 of baricitinib L-tartaric acid salt.

### Example 25

### Preparation of 2-[1-Ethylsulfonyl-3-[4-(7H-pyrrolo[2,3-d]pyrimidin-4-yl)pyrazol-1-yl]azetidin-3-yl]acetonitrile citric acid salt amorphous form

1g of baricitinib free base (2.7 mmol) was dissolved in 15 mL tetrahydrofuran by heating to 50°C applying a continuous stirring and 310 mg of citric acid (1.62 mmol) dissolved in 0.5 mL water at was drop-wise added. The solution was held at 50°C for 1 hour, then it was slowly cooled down to room temperature and held there for 2 hours. As no precipitation occurred, the solvent was evaporated till complete dryness.

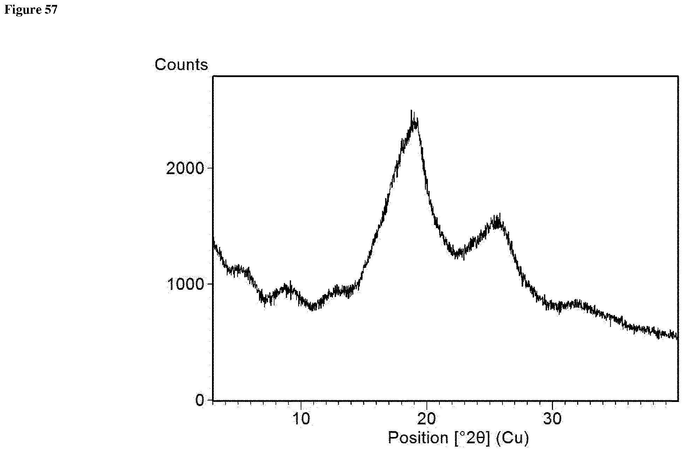

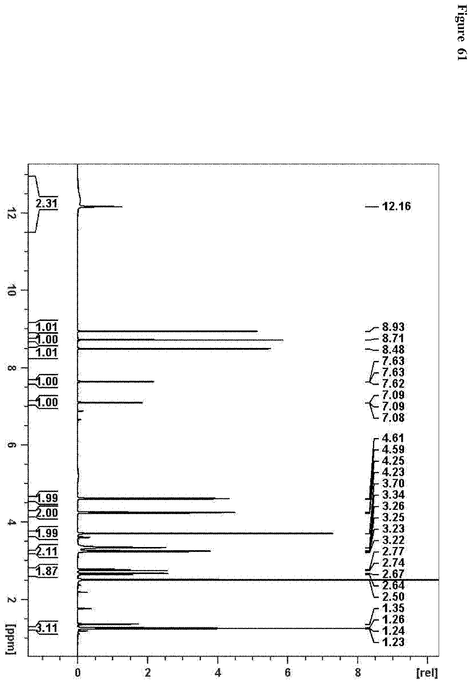

**Product:** 1.19 g (2.77 mmol) white crystalline solid, HPLC: 99.2%
The stoichiometry determined by ¹H NMR (**Figure 61**) is API: citric acid 1:0.45. XRPD pattern (**Figure 57**) was measured and showed that the compound is an essentially amorphous phase.

## Claims

1. A salt comprising 2-[1-Ethylsulfonyl-3-[4-(7H-pyrrolo[2,3-d]pyrimidin-4-yl)pyrazol-1-yl]azetidin-3-yl]acetonitrile of Formula I and at least one acid component (HX) selected from the group consisting of hydrobromic acid and hydrochloric acid, ***characterized in that*** the acid addition salt is in a crystalline form.

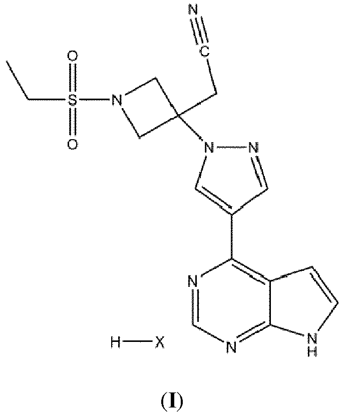

2. The crystalline salt as claimed in claim 1, ***characterized in that*** the acid component (HX) is a hydrobromic acid.

3. A Crystal modification 1 of the crystalline salt as claimed in claim 2, wherein at least one acid component (HX) is a hydrobromic acid, ***characterized in that*** an X-ray powder diffraction pattern comprises characteristic peaks at about 10.8; 16.8; 20.2; 21.1; 21.8; 25.7 and 27.8 ± 0.2° 2-theta measured by CuKα radiation.

4. A process for the preparation of the Crystal modification 1 of claim 3, wherein baricitinib free form is dissolved in a suitable organic solvent and hydrobromic acid is then added.

5. The process of claim 4, wherein the suitable organic solvent is selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably tetrahydrofuran, even more preferably the suitable organic solvent is tetrahydrofuran at the temperature of 50°C.

6. The process of claims 4 and 5, wherein the polar protic solvent is tetrahydrofuran, preferably at the temperature of 50 °C.

7. The process of claims 4 to 6, comprising the steps of:
a/ dissolution of baricitinib free in a suitable organic solvent, wherein the suitable organic solvent is selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably tetrahydrofuran, even more preferably the suitable organic solvent is tetrahydrofuran at the temperature of 50°C.
b/ drop-wise addition of the hydrobromic acid 48% aqueous solution;
c/ cooling the solution of the step b/ to a temperature of 0-25°C while precipitation occurs;
d/ keeping the suspension of the step c/ for about 16 hours at a temperature of 0-25°C;
e/ isolating the baricitinib hydrobromic acid salt in Crystal modification 1
f/ optionally, drying of the product of step e/ at laboratory condition until the constant weight of the product is reached.

8. The crystalline salt as claimed in claim 1, **characterized in that** the acid component (HX) is a hydrochloric acid.

9. A Crystal modification 1 of the crystalline salt as claimed in claim 8, wherein at least one acid component (HX) is a hydrochloric acid, ***characterized in that*** an X-ray powder diffraction pattern comprises characteristic peaks at about 5.2; 8.9; 16.0; 20.0; 21.4; 23.9 and 28.7 ± 0.2° 2-theta measured by CuKα radiation.

10. A process for the preparation of the Crystal modification 1 of claim 9, wherein baricitinib free form is dissolved in a suitable organic solvent and hydrochloric acid is then added.

11. The process of claim 10, wherein the suitable organic solvent is selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably tetrahydrofuran, even more preferably the suitable organic solvent is tetrahydrofuran at the temperature of 50°C

12. The process of claims 10 and 11, wherein the polar protic solvent is tetrahydrofuran, preferably at the temperature of 50 C.

13. The process of claims 10 to 12, comprising the steps of:
a/ dissolution of baricitinib free in a suitable organic solvent wherein the suitable organic solvent is selected from the group consisting of: C1-C6 alcohols, preferably methanol, ethanol, 1-propanol, 2-propanol, 1-butanol, 2-butanol or a mixture thereof or polar aprotic solvent, preferably acetone, acetonitrile, ethyl acetate, butyl acetate, tetrahydrofuran or a mixture thereof, more preferably tetrahydrofuran, even more preferably the suitable organic solvent is tetrahydrofuran at the temperature of 50°C;
b/ drop-wise addition of the hydrochloric acid 35% aqueous solution;
c/ cooling the solution of the step b/ to a temperature of 0-25°C while precipitation occurs;
d/ keeping the suspension of the step c/ for about 16 hours at a temperature of 0-25°C;
e/ isolating the baricitinib hydrochloric acid salt in Crystal modification 1
f/ optionally, drying of the product of step e/ at laboratory condition until the constant weight of the product is reached.

14. A Crystal modification 2 of the crystalline salt as claimed in claim 8, wherein at least one acid component (HX) is a hydrochloric acid, ***characterized in that*** an X-ray powder diffraction pattern comprises characteristic peaks at about 11.1; 17.0; 20.2; 22.0; 24.5; 25.9 and 28.2 ± 0.2° 2-theta measured by CuKα radiation.

15. A process for the preparation of the Crystal modification 2 of claim 14, wherein baricitinib hydrobromic acid salt is suspended in water.

16. The process of claim 15, wherein baricitinib hydrobromic acid salt is suspended in water at 50°C applying continuous stirring.

17. The process of claims 15 and 16, comprising the steps of:
a/ suspending baricitinib hydrobromic acid salt in water at 50°C;
b/ stirring the suspension of step a/ at room temperature for 72 hours;
c/ isolating the Crystal modification 2 of baricitinib hydrochloric acid salt;
d/ optionally, drying of the product of step c/ at laboratory condition until the constant weight of the product is reached.
